(19) Europäisches Patentamt
European Patent Office
Office européen des brevets

(11) EP 1 003 724 B1

(12) # EUROPÄISCHE PATENTSCHRIFT

(45) Veröffentlichungstag und Bekanntmachung des Hinweises auf die Patenterteilung:
**12.02.2003 Patentblatt 2003/07**

(21) Anmeldenummer: **97915425.9**

(22) Anmeldetag: **25.03.1997**

(51) Int Cl.[7]: **C07D 215/50**, A61K 31/47, C07D 401/12

(86) Internationale Anmeldenummer:
**PCT/EP97/01496**

(87) Internationale Veröffentlichungsnummer:
**WO 97/037977 (16.10.1997 Gazette 1997/44)**

(54) **SUBSTITUIERTE-CHINOLIN-DERIVATE MIT ANTIVIRALER WIRKUNG**
SUBSTITUTED QUINOLINE DERIVATIVES WITH ANTIVIRAL ACTION
DERIVES DE QUINOLEINE SUBSTITUEE A EFFET ANTIVIRAL

(84) Benannte Vertragsstaaten:
**AT BE CH DE DK ES FI FR GB GR IE IT LI LU NL PT SE**

(30) Priorität: **04.04.1996 DE 19613591**

(43) Veröffentlichungstag der Anmeldung:
**31.05.2000 Patentblatt 2000/22**

(73) Patentinhaber: **Aventis Pharma Deutschland GmbH**
**65929 Frankfurt am Main (DE)**

(72) Erfinder:
- **KIRSCH, Reinhard**
  **D-38100 Braunschweig (DE)**
- **KLEIM, Jörg-Peter**
  **65527 Niedernhausen (DE)**
- **RIESS, Günther**
  **D-65795 Hattersheim (DE)**
- **ROSENSTOCK, Bernd**
  **D-63263 Neu-Isenburg (DE)**
- **RÖSNER, Manfred**
  **D-65817 Eppstein (DE)**
- **WINKLER, Irvin**
  **D-65835 Liederbach (DE)**

(56) Entgegenhaltungen:
**EP-A- 0 579 968** **WO-A-93/11115**

- **CHEMICAL ABSTRACTS, vol. 124, no. 13, 25.März 1996 Columbus, Ohio, US; abstract no. 175786s, FUJITSA,REIKO: "Syntheses of quinolones ..." XP002033773 & ANNU. REP. TOHOKU COLL. PHARM., Bd. 41, - 1994 Seiten 113-117,**
- **CHEMICAL ABSTRACTS, vol. 121, no. 15, 10.Oktober 1994 Columbus, Ohio, US; abstract no. 179581g, SUZUKI,KOJI ET AL: "Preparation of imidazopyridines and ..." XP002033774 & JP 00 692 939 A (KYOWA HAKKO KOGYO KK)**
- **CHEMICAL ABSTRACTS, vol. 118, no. 5, 1.Februar 1993 Columbus, Ohio, US; abstract no. 38750n, HAYASHI,HIROAKI ET AL: "5-HT3 receptor antagonists. 1. New quinoline derivatives" XP002033775 in der Anmeldung erwähnt & JOURNAL OF MEDICINAL CHEMISTRY, Bd. 35, Nr. 26, - 1992 WASHINGTON US, Seiten 4893-4902,**
- **CHEMICAL ABSTRACTS, vol. 89, no. 5, 31.Juli 1978 Columbus, Ohio, US; abstract no. 43065e, MASTAFANOVA,L.I. ET AL: "Reaction of derivatives ..." XP002033776 & KHIM. GETEROTSIKL. SOEDIN., Bd. 3, - 1978 Seiten 368-373,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 003 724 B1

Printed by Jouve, 75001 PARIS (FR)

## Beschreibung

Gegenstand der vorliegenden Erfindung sind Chinolin-4-carbonsäurederivate und verwandte Verbindungen, Verfahren zu ihrer Herstellung und ihre Verwendung.

Virale Infektionen bei Mensch und Tier, insbesondere beim Menschen, sind weit verbreitet. Trotz intensiver Bemühungen ist es bislang jedoch noch nicht gelungen, Chemotherapeutika aufzufinden, die ursächlich oder symtomatisch mit dem viral oder retroviral bedingten Krankheitsgeschehen mit einem erkennbar substantiellen Erfolg interferieren. Eine Behandlung viraler und insbesondere retroviraler Erkrankungen mittels Chemotherapeutika ist daher nur sehr unvollkommen. Aufgrund der weltweit stark ansteigenden Zahl an Personen, welche mit dem HIV-Virus infiziert sind, stellt insbesondere diese Art der retroviralen Virusinfektion ein weltweit wachsendes Problem dar.

Das als humanes Immundefizienzvirus (HIV) bezeichnetes Retrovirus wird i.a. als Verursacher der komplexen Krankheit, welche man als AIDS (Acquired Immune Deficiency Syndrome) bezeichnet, angenommen. AIDS verursacht eine progressive Zerstörung des Immunsystems des Erkrankten, verbunden mit einer Zerstörung des periphären und des zentralen Nervensystems. Ein wichtiger Schritt im Replikationszyklus von Retroviren ist die reverse Transcription des RNA-Genoms des Virus durch das viruseigene Enzym Reverse Transcriptase, welche DNA-Kopien der HIV-Sequenz liefert. Es ist bekannt, daß einige Verbindungen, wie z.B. Azidothymidin (AZT), als Inhibitoren der Reversen Transcriptase fungieren können. Sie werden daher zur Behandlung von AIDS angewendet. AZT und ähnliche Verbindungen vom Nukleosidtyp wie DDC oder DDI sind jedoch durch eine sehr enge therapeutische Breite bzw. durch bereits im therapeutischen Bereich auftretende, sehr schwere Toxizitäten charakterisiert (s. z.B. Hirsch, M.S. J. Infect. Dis. 157 (1988), 427 - 431). Des weiteren ist das Problem der Resistenzbildung gegen Chemotherapeutika noch weitgehend ungelöst.

Iminochinolinderivate mit antiviraler Wirksamkeit gegen das HIV-Virus sind in der Patentanmeldung EP 93109965.9 beschrieben. Chinoxalinon-Derivate mit verwandten Strukturen mit antiviraler Wirksamkeit sind in der Patentanmeldung EP 509.398 beschrieben.

Die 4-Cyano-substituierten Chinolin-2(1H)-thione der Formeln a und b sind literaturbekannt (s. Senthil et al., Ind. J.Chem., Sect. B. 1989, 28B (12), 1017 - 209).

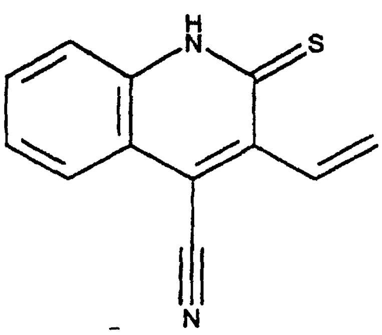

(a)

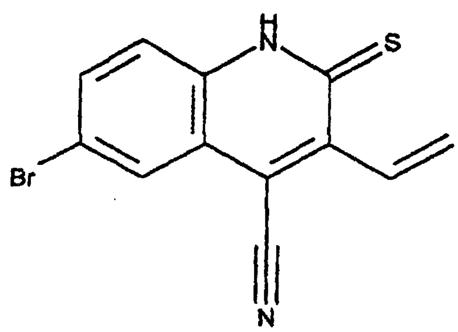

(b)

Verbindungen der allgemeinen Struktur c mit R'gleich Wasserstoff, Alkyl, Aryl oder Benzyl, R" gleich Wasserstoff, Alkyl und R''' gleich 3-Tropanol (N-8-Methyl-8-azabicyclo[3.2.1.]-octan-3-ol oder endo N-8-Methyl-8-azabicycto[3.2.1.]-oct-3-yl-amin sind als potentielle 5-$HT_3$-Rezeptorantagonisten vorgeschlagen worden (J. Med. Chem- 1992, 35, 4893 - 4902)

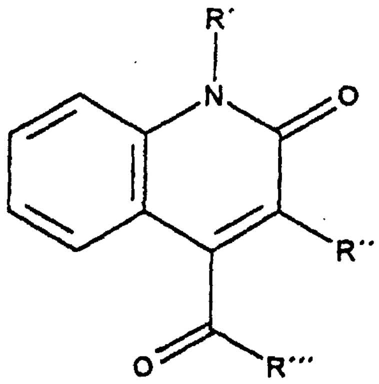

c

Die Herstellung der 2-Oxo-1,2-dihydrochinolincarbonsäure d ausgehend von Isatin und Malonsäure oder 2-Oxindol ist ebenfalls literaturbekannt (J. Am. Chem. Soc. 75 (1953), 5305). Die Darstellung von 2-Oxo-1,2-dihydro-3-ethylchinolincarbonsäure (e) aus 2-oxindol und 2 -Oxo-Buttersäure erfolgt auf ähnliche Weise ( Bull. Chem. Soc. Jpn., Vol. 50 (8) (1977), 1959 - 63).

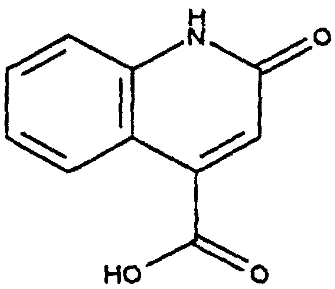

(d)

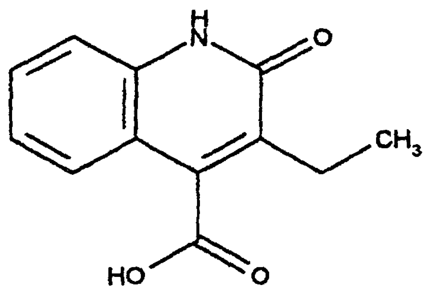

(e)

In der Patentanmeldung WO 93/11115 werden 2-(1H)-Chinolinonderivate der allgemeinen Formel f mit einem Aryl-Substituenten in 3 -Position und R gleich einem Alkoxycarbonyl-Substituenten als selektive nicht-kompetitive NMDAund/oder AMPA-Rezeptorantagonisten zur Behandlung von Krankheiten, wie z.B. neurodegenerativen Erkrankungen oder Schizophrenie, beansprucht. Eine antivirale Aktivität dieser Derivate ist jedoch nirgends erwähnt.

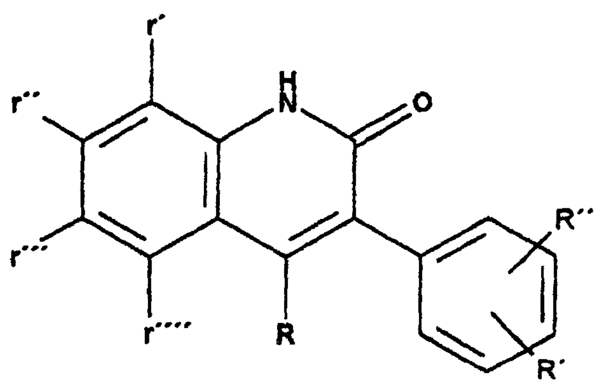

f

Es wurde nun überraschenderweise gefunden, daß bestimmte Chinolinderivate eine hohe antivirale Wirksamkeit, insbesondere gegen das Humane Immundefizienzvirus (HIV) aufweisen.
Erfindungsgegenstand sind demzufolge Verbindungen der Formel I,

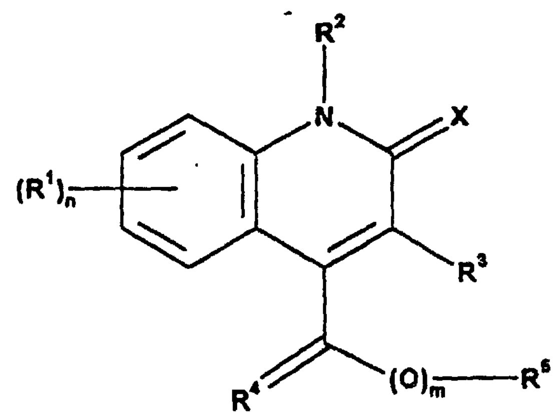

(I)

sowie deren tautomere Formen, der allgemeinen Formel Ia,

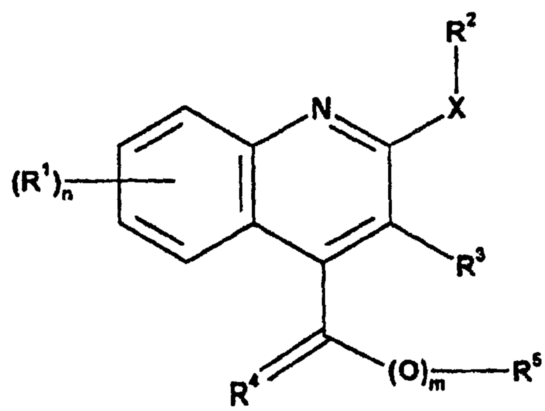

(Ia)

worin bedeuten:
IV)
n
null,
eins,
zwei,
die einzelnen Substituenten R1 unabhängig voneinander
Fluor, Chlor, Brom, Trifluormethyl, Trifluormethoxy, $(C_1-C_6)$-Alkyl, $(C_3-C_6)$-Cycloalkyl, $(C_1-C_6)$-Alkoxy, $(C_1-C_6)$-Alkoxy($(C_1-C_6)$-alkoxy), $(C_1-C_6)$-Alkylthio, $(C_1-C_6)$-Alkylsulfinyl, $(C_1-C_6)$-Acyl, $(C_1-C_6)$-Acyloxy, Carboxy, $(C_1-C_6)$-Alkyloxycarbonyl,
oder
einen gegebenenfalls mit bis zwei voneinander unabhängigen Resten $R^6$ substituierten Phenyl-, Phenoxy-, Phenoxycarbonyl-, Phenylthio-, Phenylsulfonyl-, Phenoxysulfonyl-, Phenylsulfonyloxy-, Phenylsulfonylamino-, Benzoylrest, wobei $R^6$
Fluor, Chlor, Brom, Trifluormethyl, Trifluormethoxy, Azido, $(C_1-C_4)$-Alkyl, $(C_4-C_6)$-Cycloalkyl, $(C_1-C_4)$-Alkoxy, $(C_1-C_4)$ -Alkylthio sein kann,
X Sauerstoff, Schwefel oder substituierten Stickstoff N-$R^2$, N-O-$R^2$, worin $R^2$ die unten gegebenen Bedeutungen haben kann,
und $R^2$ Wasserstoff,

$(C_1-C_4)$-Alkyl, gegebenenfalls substituiert mit Fluor, Chlor, Brom; $(C_1-C_3)$-Acyloxy, Benzoyloxy, Benzyloxy, Phenoxy, $(C_1-C_3)$-Alkoxy, Phenylsulfonyl, Oxo, Carboxy, Carbamoyl;
$(C_2-C_6)$-Alkenyl, gegebenenfalls substituiert mit Fluor, Chlor, Brom, $(C_1-C_3)$-Acyloxy, Benzoyloxy, Benzyloxy, Phenoxy, $(C_1-C_3)$-Alkoxy, Phenylsulfonyl, Oxo, Carboxy, Carbamoyl;
und
$R^3$
$(C_1-C_8)$-Alkyl, gegebenenfalls substituiert mit Fluor, Chlor, Hydroxy, Amino, $(C_1-C_4)$-Acyloxy, Benzoyloxy, Benzyloxy, Phenoxy, $(C_1-C_4)$-Alkoxy, Carboxy;
$(C_2-C_6)$-Alkenyl, gegebenenfalls substituiert mit Fluor, Chlor, Hydroxy, Amino, $(C_1-C_4)$-Acyloxy, Benzoyloxy, Benzyloxy, Phenoxy, $(C_1-C_4)$-Alkoxy, Carboxy;
$(C_3-C_6)$-Cycloalkyl, gegebenenfalls substituiert ist Fluor, Chlor, Hydroxy, Amino, $(C_1-C_4)$-Acyloxy, Benzoyloxy, Benzyloxy, Phenoxy, $(C_1-C_4)$-Alkoxy, Carboxy;
$(C_3-C_6)$-Cycloalkenyl, gegebenenfalls substituiert ist Fluor, Chlor, Hydroxy, Amino, $(C_1-C_4)$-Acyloxy, Benzoyloxy, Benzyloxy, Phenoxy, $(C_1-C_4)$-Alkoxy, Carboxy;
oder unsubstituiertes oder mit bis zu 2 voneinander unabhängigen Resten $R^6$ substituiertes Aryl, Aryl-$(C_1-C_2)$-alkyl bedeuten, wobei $R^6$ wie oben definiert ist,
und $R^4$ Sauerstoff,
und m gleich 0 oder 1,
und $R^5$
$(C_1-C_8)$-Alkyl, gegebenenfalls substituiert mit Fluor, Chlor, Brom, Cyano, Amino, Hydroxy, $(C_1-C_4)$-Acyloxy, Benzoyloxy, Benzyloxy, Phenoxy, $(C_1-C_4)$-Alkoxy, Oxo, Carboxy, Carbamoyl;
$(C_2-C_8)$-Alkenyl, gegebenenfalls substituiert mit Fluor, Chlor, Brom, Jod, Cyano, Amino, Hydroxy, $(C_1-C_4)$-Acyloxy, Benzoyloxy, Benzyloxy, Phenoxy, $(C_1-C_4)$-Alkoxy, Oxo, Carboxy, Carbamoyl;
$(C_2-C_6)$-Alkinyl, gegebenenfalls substituiert mit Fluor, Chlor, Brom, Cyano, Amino, Hydroxy, $(C_1-C_4)$-Acyloxy, Benzoyloxy, Benzyloxy, Phenoxy, $(C_1-C_4)$-Alkoxy, Oxo, Carboxy, Carbamoyl;
$(C_3-C_6)$-Cycloalkyl, gegebenenfalls substituiert mit Fluor, Chlor, Brom, Cyano, Amino, Hydroxy, $(C_1-C_4)$-Acyloxy, Benzoyloxy, Benzyloxy, Phenoxy, $(C_1-C_4)$-Alkoxy, Oxo, Carboxy, Carbamoyl;
$(C_3-C_6)$-Cycloalkenyl, gegebenenfalls substituiert mit Fluor, Chlor, Brom, Amino, Hydroxy, $(C_1-C_4)$-Acyloxy, Benzoyloxy, Benzyloxy, Phenoxy, $(C_1-C_4)$-Alkoxy;
$(C_3-C_6)$-(Cycloalkyl)-($(C_1-C_2)$-alkyl), gegebenenfalls substituiert mit Fluor, Chlor, Brom, Amino, Hydroxy, $(C_1-C_4)$-Acyloxy, Benzoyloxy, Benzyloxy, Phenoxy;
$(C_3-C_6)$-(Cycloalkenyl)-($(C_1-C_2)$-alkyl), gegebenenfalls substituiert mit Fluor, Chlor, Brom, Amino, Hydroxy, $(C_1-C_4)$-Acyloxy, Benzoyloxy, Benzyloxy, Phenoxy;
oder unsubstituiertes oder mit bis zu zwei voneinander unabhängigen Resten $R^6$ substituiertes Aryl, Aryl-$(C_1-C_4)$-alkyl, Aryl-$(C_2-C_4)$-alkenyl, wobei $R^6$ wie oben definiert ist;
oder unsubstituiertes oder mit bis zu drei voneinander unabhängigen Resten $R^6$ substituiertes Heteroaryl, Heteroaryl-$(C_1-C_4)$-alkyl,
deren optische Isomere, Diastereomere in reiner Form oder in Form ihrer Mischungen und deren Additionsalze und Prodrugs
mit Ausnahme der Verbindungen der Formel I, in denen gleichzeitig die Reste $R^1$,$R^2$, $R^3$, $R^4$, $R^5$, X und m die folgenden Bedeutungen aufweisen:

$R^2$ gleich Wasserstoff,
$R^3$ gleich unsubstituiertes oder wie oben angegeben substituiertes Phenyl,
X gleich Sauerstoff,
$R^4$ gleich Sauerstoff,
m gleich 1 und
$R^5$ gleich $C_1-C_6$-Alkyl

weiterhin mit Ausnahme der Verbindungen der Formel I, in denen gleichzeitig die Reste $R^2$, $R^3$, $R^4$, $R^5$, X und m die folgenden Bedeutungen aufweisen:

$R^2$ gleich Wasserstoff,
$R^3$ gleich unsubstituiertes oder wie oben angegeben substituiertes Phenyl,
X gleich Sauerstoff,
$R^4$ gleich Sauerstoff,
m gleich O und
$R^5$ wie oben definiert.

In einer bevorzugten Gruppe von Verbindungen der Formel I und Ia bedeuten;

V) n

null, eins, der Substituenten $R^1$

Fluor, Chlor, Brom, Trifluormethyl, Trifluormethoxy, $(C_5-C_6)$-Cycloalkyl, $(C_1-C_4)$-Alkoxy, $(C_1-C_4)$-Alkoxy($(C_1-C_4)$-alkoxy), $(C_1-C_4)$-Alkylthio, $(C_1-C_4)$-Acyl,

oder

einen gegebenenfalls mit einem Rest $R^6$ substituierten Phenyl-, Phenoxy-, Benzoylrest,

wobei $R^6$

Fluor, Chlor, Brom, Trifluormethyl, Trifluormethoxy, $(C_1-C_4)$-Alkyl, $(C_1-C_4)$-Alkoxy, $(C_1-C_4)$-Alkylthio, sein kann,

X Sauerstoff oder Schwefel,

und $R^2$ Wasserstoff,

und

$R^3$

$(C_1-C_6)$-Alkyl;

$(C_2-C_6)$-Alkenyl;

$(C_3-C_6)$-Cycloalkyl;

oder unsubstituiertes oder mit bis zu 2 voneinander unabhängigen Resten $R^6$ substituiertes Phenyl, Benzyl, wobei $R^6$ wie oben definiert ist,

und $R^4$ Sauerstoff,

und m gleich 1,

und $R^5$

$(C_1-C_6)$-Alkyl, gegebenenfalls substituiert mit Fluor, Chlor, Brom, Cyano, Amino, Hydroxy, $(C_1-C_4)$-Acyloxy, Benzoyloxy, Benzyloxy, Phenoxy, $(C_1-C_4)$-Alkoxy;

$(C_2-C_6)$-Alkenyl, gegebenenfalls substituiert mit Fluor, Chlor, Brom, Amino, Hydroxy, $(C_1-C_4)$-Acyloxy, Carboxy;

$(C_3-C_6)$-Cycloalkyl;

$(C_3-C_6)$-Cycloalkenyl;

$(C_3-C_6)$-(Cycloalkyl)-($(C_1-C_2)$-alkyl);

oder unsubstituiertes oder mit bis zu zwei voneinander unabhängigen Resten $R^6$ substituiertes Aryl, Aryl-$(C_1-C_4)$-alkyl, wobei $R^6$ wie oben definiert ist;

deren optische Isomere, Diastereomere in reiner Form oder in Form ihrer Mischungen und deren Additionsalze und Prodrugs.

VI) Ganz besonders bevorzugt sind die in der nachfolgenden Tabelle T. 1 aufgeführten, beispielhaft genannten Verbindungen:

Tabelle T1:

| Beispiele ganz besonders bevorzugter Verbindungen | | | | | | | |
|---|---|---|---|---|---|---|---|
| Verbindungs nummer | n | $R^1$ | $R^2$ | $R^3$ | $R^4$ | $(O)_m-R^5$ | X |
| a.1 | 1 | 6-Cl | H | methyl | O | O-ethyl | O |
| a.2 | 1 | 6-Cl | H | methyl | O | O-ethyl | S |
| a.3 | 1 | 6-Cl | H | methyl | O | O-ethyl | NOH |
| a.4 | 1 | 6-Cl | H | methyl | O | O-isopropyl | O |
| a.5 | 1 | 6-Cl | H | methyl | O | O-isopropyl | S |
| a.6 | 1 | 6-Cl | H | methyl | O | O-isopropyl | NOH |
| a.7 | 1 | 6-OMe | H | methyl | O | O-ethyl | O |
| a.8 | 1 | 6-OMe | H | methyl | O | O-ethyl | S |
| a.9 | 1 | 6-OMe | H | methyl | O | O-ethyl | NOH |
| a.10 | 1 | 6-OMe | H | methyl | O | O-isopropyl | O |
| a.11 | 1 | 6-OMe | H | methyl | O | O-isopropyl | S |
| a.12 | 1 | 6-OMe | H | methyl | O | O-isopropyl | NOH |
| a.13 | 1 | F | H | methyl | O | O-ethyl | O |

Tabelle T1: (fortgesetzt)

| Beispiele ganz besonders bevorzugter Verbindungen | | | | | | | |
|---|---|---|---|---|---|---|---|
| Verbindungs nummer | n | $R^1$ | $R^2$ | $R^3$ | $R^4$ | $(O)_m$-$R^5$ | X |
| a.14 | 1 | F | H | methyl | O | O-ethyl | S |
| a.15 | 1 | F | H | methyl | O | O-ethyl | NOH |
| a.16 | 1 | F | H | methyl | O | O-isopropyl | O |
| a. 17 | 1 | F | H | methyl | O | O-isopropyl | S |
| a.18 | 1 | F | H | methyl | O | O-isopropyl | NOH |
| a.19 | 1 | 6-Cl | H | ethyl | O | O-ethyl | O |
| a.20 | 1 | 6-Cl | H | ethyl | O | O-ethyl | S |
| a.21 | 1 | 6-OMe | H | ethyl | O | O-ethyl | O |
| a.22 | 1 | 6-OMe | H | ethyl | O | O-ethyl | S |
| a.23 | 1 | F | H | ethyl | O | O-ethyl | O |
| a.24 | 1 | F | H | ethyl | O | O-ethyl | S |
| a.25 | 1 | 6-Cl | H | ethyl | O | O-isopropyl | O |
| a.26 | 1 | 6-Cl | H | ethyl | O | O-isopropyl | S |
| a.27 | 1 | 6-OMe | H | ethyl | O | O-isopropyl | O |
| a.28 | 1 | 6-OMe | H | ethyl | O | O-isopropyl | S |
| a.29 | 1 | F | H | ethyl | O | O-isopropyl | O |
| a.30 | 1 | F | H | ethyl | O | O-isopropyl | S |
| a.31 | 1 | 6-Cl | H | isopropyl | O | O-ethyl | O |
| a.32 | 1 | 6-Cl | H | isopropyl | O | O-ethyl | S |
| a.33 | 1 | 6-OMe | H | isopropyl | O | O-ethyl | O |
| a.34 | 1 | 6-OMe | H | isopropyl | O | O-ethyl | S |
| a.35 | 1 | F | H | isopropyl | O | O-ethyl | O |
| a.36 | 1 | F | H | isopropyl | O | O-ethyl | S |
| a.37 | 1 | 6-Cl | H | isopropyl | O | O-isopropyl | O |
| a.38 | 1 | 6-Cl | H | isopropyl | O | O-isopropyl | S |
| a.39 | 1 | 6-OMe | H | isopropyl | O | O-isopropyl | O |
| a.40 | 1 | 6-OMe | H | isopropyl | O | O-isopropyl | S |
| a.41 | 1 | F | H | isopropyl | O | O-isopropyl | O |
| a.42 | 1 | F | H | isopropyl | O | O-isopropyl | S |
| a.43 | 1 | 6-Cl | H | Phenyl | O | O-ethyl | O |
| a.44 | 1 | 6-Cl | H | phenyl | O | O-ethyl | S |
| a.45 | 1 | 6-OMe | H | phenyl | O | O-ethyl | O |
| a.48 | 1 | 6-OMe | H | phenyl | O | O-ethyl | S |
| a.47 | 1 | F | H | phenyl | O | O-ethyl | O |
| a.48 | 1 | F | H | phenyl | O | O-ethyl | S |
| a.49 | 1 | 6-Cl | H | phenyl | O | O-isopropyl | O |
| a.50 | 1 | 6-Cl | H | phenyl | O | O-isopropyl | S |

Tabelle T1: (fortgesetzt)

| Beispiele ganz besonders bevorzugter Verbindungen | | | | | | | |
|---|---|---|---|---|---|---|---|
| Verbindungs nummer | n | $R^1$ | $R^2$ | $R^3$ | $R^4$ | $(O)_m$-$R^5$ | X |
| a.51 | 1 | 6-OMe | H | phenyl | O | O-isopropyl | O |
| a.52 | 1 | 6-OMe | H | phenyl | O | O-isopropyl | S |
| a.53 | 1 | F | H | propyl | O | O-isopropyl | O |
| a.54 | 1 | F | H | propyl | O | O-isopropyl | S |
| a.55 | 1 | 6-Cl | H | propyl | O | O-ethyl | O |
| a.56 | 1 | 6-Cl | H | propyl | O | O-ethyl | S |
| a.57 | 1 | 6-OMe | H | propyl | O | O-ethyl | O |
| a.58 | 1 | 6-OMe | H | propyl | O | O-ethyl | S |
| a.59 | 1 | F | H | propyl | O | O-ethyl | O |
| a.60 | 1 | F | H | propyl | O | O-ethyl | S |
| a.61 | 1 | 6-Cl | H | propyl | O | O-isopropyl | O |
| a.62 | 1 | 6-Cl | H | propyl | O | O-isopropyl | S |
| a.63 | 1 | 6-OMe | H | propyl | O | O-isopropyl | O |
| a.64 | 1 | 6-OMe | H | propyl | O | O-isopropyl | S |
| a.65 | 1 | F | H | propyl | O | O-isopropyl | O |
| a.66 | 1 | F | H | propyl | O | O-isopropyl | S |
| a.67 | 1. | 6-Cl | H | n-butyl | O | O-ethyl | O |
| a.68 | 1 | 6-Cl | H | n-butyl | O | O-ethyl | S |
| a.69 | 1 | 6-OMe | H | n-butyl | O | O-ethyl | O |
| a.70 | 1 | 6-OMe | H | n-butyl | O | O-ethyl | S |
| a.71 | 1 | F | H | n-butyl | O | O-ethyl | O |
| a.72 | 1 | F | H | n-butyl | O | O-ethyl | S |
| a.73 | 1 | 6-Cl | H | n-butyl | O | O-isopropyl | O |
| a.74 | 1 | 6-Cl | H | n-butyl | O | O-isopropyl | S |
| a.75 | 1 | 6-OMe | H | n-butyl | O | O-isopropyl | O |
| a.76 | 1 | 6-OMe | H | n-butyl | O | O-isopropyl | S |
| a.77 | 1 | F | H | n-butyl | O | O-isopropyl | O |
| a.78 | 1 | F | H | n-butyl | O | O-isopropyl | S |
| a.79 | 1 | H | H | methyl | O | O-isopropyl | O |
| a.80 | 1 | H | H | methyl | O | O-isopropyl | S |
| a.81 | 1 | H | H | ethyl | O | O-isopropyl | O |
| a.82 | 1 | H | H | ethyl | O | O-isopropyl | S |
| a.83 | 1 | H | H | isopropyl | O | O-isopropyl | O |
| a.84 | 1 | H | H | isopropyl | O | O-isopropyl | S |
| a.85 | 1 | H | H | methyl | O | O-ethyl | O |
| a.86 | 1 | H | H | methyl | O | O-ethyl | S |
| a.87 | 1 | H | H | ethyl | O | O-ethyl | O |

Tabelle T1: (fortgesetzt)

| Beispiele ganz besonders bevorzugter Verbindungen | | | | | | | |
|---|---|---|---|---|---|---|---|
| Verbindungs nummer | n | $R^1$ | $R^2$ | $R^3$ | $R^4$ | $(O)_m$-$R^5$ | X |
| a.88 | 1 | H | H | ethyl | O | O-ethyl | S |
| a.89 | 1 | H | H | isopropyl | O | O-ethyl | O |
| a.90 | 1 | H | H | isopropyl | O | O-ethyl | S |

Bevorzugt sind weiterhin Verbindungen der Formel I, in denen mindestens einer der Substituenten unabängig voneinander die folgende Bedeutung besitzt:

n gleich null oder eins;

der Substituent $R^1$
Fluor, Chlor, Brom, Trifluormethoxy, Methoxy, Ethoxy, Propoxy, Butoxy, Methylthio, Ethylthio, Propylthio, Butylthio, Methoxymethoxy, Methoxyethoxy, Acetyl, Propionyl,
oder
einen gegebenenfalls mit einem Rest $R^6$ substituierten Phenyl, Phenoxy oder Benzoylrest,
wobei $R^6$
Fluor, Chlor, Brom, Trifluormethyl, Trifluormethoxy, Methyl, Ethyl, Propyl, Methoxy, Ethoxy, Propoxy, Methylthio oder Ethylthio sein kann;

X Sauerstoff oder Schwefel;
$R^2$ Wasserstoff;
$R^3$ Methyl, Ethyl, Propyl, Butyl, Pentyl oder Hexyl, Ethenyl, Propenyl, Butenyl, Pentenyl, Hexenyl, Cyclopropyl, Cyclobutyl, Cyclopentyl, Cyclohexyl;
$R^3$ unsubstituiertes oder mit bis zu zwei voneinander unabhängigen Resten $R^6$ substituiertes Phenyl oder Benzyl, wobei $R^6$ wie oben definiert ist;
$R^4$ gleich Sauerstoff oder Schwefel;
m gleich null oder eins;
und
$R^5$ gleich Methyl, Ethyl, Propyl, Butyl, Pentyl, Hexyl, Ethenyl, Propenyl, Butenyl, Pentenyl, Hexenyl, Cyclopropyl, Cyclobutyl, Cyclopentyl, Cyclohexyl, Cyclopropylmethyl, Cyclobutylmethyl, Cyclopentylmethyl, Cyclohexylmethyl, oder $R^5$ gleich unsubstituiertes oder mit bis zu zwei voneinander unabhängigen Resten $R^6$ substituiertes Phenyl, Benzyl, Phenylethyl, wobei $R^6$ wie oben definiert ist.

Erfindungsgegenstand ist weiterhin die Verwendung von Verbindungen der Formeln I und I a zur Herstellung von Arzneimitteln zur Behandlung oder Prophylaxe von retroviralen Erkrankungen wie zum Beispiel AIDS.

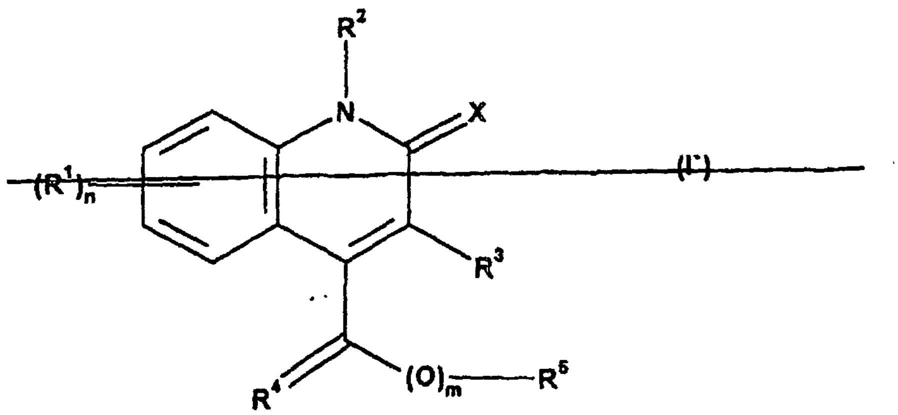

Die in den vorangegangenen Definitionen genannten Alkylgruppen können geradkettig oder verzweigt sein.

Sofern nicht anders definiert, enthalten sie vorzugsweise 1-8, besonders bevorzugt 1-6, insbesondere 1-4 C-Atome. Beispiele sind die Methyl-, Ethyl-, Propyl-, 1-Methylethyl-, Butyl-, 1-Methylpropyl-, 2-Methylpropyl-, 1,1-Dimethylethylgruppe und ähnliche.

Die Alkylreste von Dialkylaminogruppen können gleich oder verschieden sein und sind insbesondere wie oben angegeben definiert. Die Angabe zur Anzahl der Kohlenstoffatome in den Dialkylverbindungen bezieht sich immer nur auf einen der Alkylreste.

Die in den vorangegangenen Definitionen genannten Alkenylgruppen können geradkettig oder verzweigt sein und enthalten 1 bis 3 Doppelbindungen. Sofern nicht anders definiert, enthalten diese Gruppen vorzugsweise 2-8, insbesondere 2-6 C-Atome. Beispiele sind die 2-Propenyl-, 1-Methylethenyl-, 2-Butenyl-, 3-Butenyl-, 2-Methyl-2-propenyl-, 3-Methyl-2-butenyl-, 2,3-Dimethyl-2-butenyl-, 3,3-Dichlor-2-propenyl- und Pentadienylgruppe und ähnliche.

Die in den vorangegangenen Definitionen genannten Alkinylgruppen können geradkettig oder verzweigt sein und enthalten 1 bis 3 Dreifachbindungen. Sofern nicht anders definiert, enthalten sie vorzugsweise 2-8, besonders bevorzugt 3-6 C-Atome. Beispiele sind die 2-Propinyl- und 3-Butinylgruppe und ähnliche.

Die in den vorangegangenen Definitionen genannten Cycloalkyl- und Cycloalkenylgruppen enthalten, sofern nicht anders definiert, vorzugsweise 3-8, besonders bevorzugt 4-6 C-Atome. Beispiele sind die Cyclopropyl-, Cyclobutyl-, Cyclopentyl-, Cyclopentenyl-, Cyclohexyl- oder Cyclohexenylgruppe.

Die in den vorangegangenen Definitionen genannten Acylgruppen können aliphatisch, cycloaliphatisch oder aromatisch sein. Sofern nicht anders definiert, enthalten sie vorzugsweise 1-8, besonders bevorzugt 2-7 C-Atome. Beispielhafte Acylgruppen sind die Formyl-, Acetyl-, Chtoracetyl-, Trifluoracetyl-, Hydroxyacetyl-, Glycyl-, Propionyl-, Butyryl-, Isobutyryl-, Pivaloyl-, Cyclohexanoyl- oder Benzoylgruppe.

Für die in den vorangegangenen Definitionen genannten Arylgruppen sind vorzugsweise aromatische Gruppen mit 6-14 C-Atomen geeignet, insbesondere mit 6-10 C-Atomen wie zum Beispiel Phenyl und Naphthyl.

In den obengenannten heterocyclischen Ringen bzw. Heteroarylgruppen kommen als Heteroatome insbesondere zum Beispiel O, S, N in Betracht, wobei im Falle eines an dieser Stelle gesättigten N-haltigen Ringes N-Z vorliegt, worin Z, H oder $R^2$ mit den jeweiligen oben beschriebenen Definitionen bedeutet.

Soweit nicht anders definiert, haben die heterocyclischen Ringe vorzugsweise 1-15 C-Atome und 1-6 Heteroatome, insbesondere 3-11 C-Atome und **1-4** Heteroatome.

Für die in den vorangegangenen Definitionen genannten heterocyclischen Ringe bzw. Heteroarylgruppen kommen beispielsweise Thiophen, Furan, Pyridin, Pyrimidin, Indol, Chinolin, Isochinolin, Oxazol, Isoxazol, Thiazol oder Isothiazol in Frage.

In gleicher Weise gelten diese Definitionen für Heteroaryl in dem Heteroarylmethylrest, Die in den vorausgegangenen Definitionen aufgeführten Aralkylgruppen sind beispielsweise Benzyl, Phenylethyl, Naphthylmethyl oder Styryl.

Die obengenannten Substituenten R1 bis R6 sind vorzugsweise 3-fach, besonders bevorzugt 2-fach, insbesondere einfach mit den jeweils angegebenen Substituenten substituiert.

Für die jeweiligen zusammengesetzten Substituentendefinitionen (wie z. B. Arylalkoxycarbonyl) sind die zuvor als bevorzugt beschriebenen Bereiche für die einzelnen Substituenten ebenfalls bevorzugt.

In Abhängigkeit von den verschiedenen Substituenten können Verbindungen der Formeln I und la mehrere asymmetrische Kohlenstoffatome besitzen. Gegenstand der Erfindung sind deshalb sowohl die reinen Stereoisomeren als auch Mischungen derselben, wie z. B. das zugehörige Racemat.

Die reinen Stereoisomeren der Verbindungen der Formeln I und la lassen sich durch bekannte Methoden oder in Analogie zu bekannten Methoden.direkt herstellen oder nachträglich trennen.

Zum Gegenstand der vorliegenden Erfindung gehören weiterhin Verfahren zur Herstellung von Verbindungen der Formeln I und la wie oben unter IV) bis VI) erläutert, dadurch gekennzeichnet, daß man

A) zur Herstellung von Verbindungen der Formel I oder la, worin X und R4 Sauerstoff, m gleich 0 oder 1, mit den übrigen Resten wie oben unter IV) bis VI) definiert, eine Verbindung der Formel II, wobei A eine Abgangsgruppe, vorzugsweise ein Halogenatom darstellt, umsetzt mit einer Verbindung der Formel III, wobei M Wasserstoff,ein Metallatom oder ein Metallatomäquivalent, vorzugsweise ein Alkali- oder Erdalkalimetall darstellt,

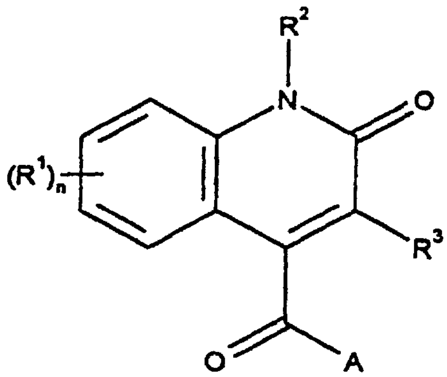

II

$$R^5 — (O)_m — M$$

III,

oder daß man
B) Verbindungen der Formel I mit X gleich Sauerstoff, und $R^1$ bis $R^5$ wie oben definiert, umsetzt mit einem Schwefelungsreagenz zu Verbindungen der Formel I mit X gleich Schwefel und $R^1$ bis $R^5$ wie oben definiert, oder daß man oder daß man
C) eine Verbindung der Formel I, mit $R^1$ - $R^5$ wie unter IV) bis VI) definiert und X ein Sauerstoff- oder Schwefelatom ist, umsetzt mit einer Verbindung der Formel IV oder IVa

$$R^2\text{-}NH_2 \qquad \text{oder} \qquad R^2\text{-}O\text{-}NH_2$$

(IV) (IVa)

zu Derivaten der Formel I mit $R^1$ - $R^5$ wie unter I) bis VI) definiert und X gleich N-$R^2$ oder N-O-$R^2$
oder daß man
D) eine Verbindung der Formel II mit A gleich Chlor und $R^1$ bis $R^5$ wie oben definiert umsetzt mit einem Reduktionsmittel zu Verbindungen der Formel I mit $R^1$ bis $R^3$ wie oben definiert, wobei $R^4$ gemeinsam mit dem verknüpfenden C-Atom eine $CH_2$-Gruppe bildet und $(O)_m$- $R^5$ gleich Hydroxy ist,

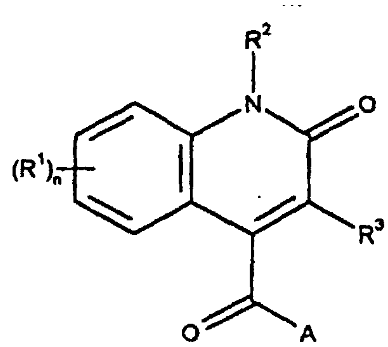

II

oder daß man
E) eine Verbindung der Formel I oder Ia mit $R^2$ gleich Wasserstoff und $R^1$, $R^3$, $R^4$ und $R^5$ wie unter IV bis VI definiert, umsetzt mit einer Verbindung der Formel V

$$R(2)\text{-}B \qquad (V),$$

wobei B eine Abgangsgruppe ist, zu Derivaten der Formel I, bei denen für R2 die in IV) bis VI) für die Verbindungen der Formel I und Ia beschriebenen Definitionen mit Ausnahme von R2 gleich Wasserstoff gelten, umsetzt und wobei Gruppen gegebenenfalls nach Verfahren des Standes der Technik in unter Y- VI genannte funktionelle Gruppen umgewandelt werden können.

Die obengenannte Methode A verläuft vorzugsweise unter folgenden Bedingungen ab:

Die Reaktion verläuft vorzugsweise mit einem 0,2 bis 10fachen Überschuß der Reaktionskomponente III. Die Reaktion wird zweckmäßigerweise in einem organischen Lösungsmittel oder einem Lösungsmittelgemisch, bei -78°C bis 150°C, bevorzugt bei der Siedetemperatur des Reaktionsgemisches und möglichst unter wasserfreien Bedingungen durchgeführt.

Als Lösungsmittel geeignet sind z. B. aromatische Kohlenwasserstoffe wie Toluol, Xylol, Ether wie Tetrahydrofuran oder Glycoldimethylether oder Gemische dieser Lösungsmittel, und weiterhin für Verbindungen mit m = 1 der zur Verbindung der Formel III korrespondierende Alkohol (M gleich Wasserstoff).

Die obengenannte Methode B verläuft vorzugsweise unter folgenden Bedingungen ab:

Für die Umsetzung wie zuvor unter B) beschrieben wird vorzugsweise als Schwefelungsreagenz 2,4-Bis(4-methoxyphenyl)-1,3-dithia-2,4-diphosphetan-2,4-disulfid (Lawesson's Reagenz), Bis(tricyclohexylzinn)sulfid,

Bis(tri-n-butylzinn)sulfid, Bis(triphenylzinn)sulfid, Bis(trimethylsilyl)sulfid oder Phosphorpentasulfid verwendet. Die Reaktion wird zweckmäßigerweise in einem organischen Lösungsmittel oder einem Lösungsmittelgemisch, bei Raumtemperatur oder höher, bevorzugt bei der Siedetemperatur des Reaktionsgemisches und möglichst unter wasserfreien Bedingungen durchgeführt. Geeignet sind z. B. Schwefelkohlenstoff, Toluol, Xylol, Pyridin oder 1,2-Dichlorethan. Bei Verwendung der erwähnten Zinn- oder Silylsulfide ist es angebracht, die Schwefelungsreaktion in Gegenwart einer Lewissäure wie Bortrichlorid durchzuführen.

In Gegenwart anderer Carbonylgruppen, sind diese gegebenenfalls vor der Schwefelungsreaktion nach bekannten Methoden durch eine geeignete Schutzgruppe, z. B. durch Acetalisierung, zu schützen.

Die obengenannte Methode C verläuft vorzugsweise unter folgenden Bedingungen ab:

Die Reaktion erfolgt durch Umsetzung einer Verbindung der Formel I, wobei I wie unter C) definiert ist, mit einem oder mehreren Moläquivalenten einer Verbindung der Formel IV, vorzugsweise 1,5 bis 4 Moläquivalenten. Gegebenenfalls können auch Salze der Verbindungen der Formel IV, vorzugsweise Salze von Halogenwasserstoffsäuren, insbesondere Salzsäure verwendet werden.

Die Reaktion kann ohne weitere Komponenten oder unter Säure- oder Basenzusatz durchgeführt werden. Insbesondere kann bei Einsatz eines Salzes einer Verbindung der Formel IV ein Äquivalent einer Base zur Freisetzung des freien Amins bzw. Hydroxylamins verwendet werden.

Die Umsetzung wird zweckmäßigerweise in einem organischen Lösungsmittel durchgeführt. Geeignet sind z. B. aromatische Kohlenwasserstoffe wie Toluol oder Xylol, niedere Alkohole wie Methanol, Ethanol, Methylglycol oder 1-Butanol, Ether wie Tetrahydrofuran oder Glycoldimethylether, basische Lösungsmittel wie Pyridin oder N-Methylimidazol oder Gemische dieser Lösungsmittel.

Die Reaktionstemperatur kann zwischen 0 und 200°C liegen, vorzugsweise zwischen 20°C und dem Siedepunkt des verwendeten Lösungsmittels bzw. Lösungsmittelgemisches

Die oben genannte Methode D läuft vorzugsweise unter folgenden Bedingungen ab.

Die Reaktion erfolgt durch Umsetzung einer Verbindung der Formel 11 mit einer stöchiometrischen Menge oder einem Überschuß eines Reduktionsmittels. Als Reduktionsmittel können insbesondere komplexe Metallhydride wie z.B. $NaBH_4$, $LiBH_4$ oder $LiAlH_4$ verwendet werden.

Die Umsetzung erfolgt zweckmäßigerweise in einem inerten organischen Lösungsmittel. Geeignet sind z.B. je nach verwendeten Reduktionsmitteln aromatische Kohlenwasserstoffe wie Toluol oder Xylol, niedere Alkohole wie Methanol oder Ethanol, Ether wie Tetrahydrofuran oder Glycoldimethylether, oder Gemische dieser Lösungsmittel.

Die Reaktionstemperatur kann zwischen 0°C und 200°C liegen, vorzugsweise zwischen 0°C und dem Siedepunkt des verwendten Lösungsmittels.

Die obengenannte Methode E verläuft vorzugsweise unter folgenden Bedingungen ab:

Die Umsetzung wird zweckmäßigerweise in einem Lösungsmittel durchgeführt. Geeignet sind z. B. aromatische Kohlenwasserstoffe wie Toluol oder Xylol, Wasser, niedere Alkohole wie Methanol, Ethanol, Methylglycol oder 1-Butanol, Ether wie Tetrahydrofuran oder Glycoldimethylether.

Die Reaktionstemperatur kann zwischen 0 und 200°C liegen, vorzugsweise zwischen 20°C und dem Siedepunkt des verwendeten Lösungsmittels bzw. Lösungsmittelgemisches.

Die obengenannte Methode F verläuft vorzugsweise unter folgenden Bedingungen ab:

Der Substituent B in der Formel V ist eine geeignete Abgangsgruppe, wie z. B. Chlor, Brom oder Jod, ein geeignetes Radikal der Schwefelsäure, ein aliphatischer oder aromatischer Sulfonsäureester oder ggf. halogeniertes Acyloxy.

Die Reaktion wird zweckmäßigerweise in einem Lösungsmittel in Gegenwart einer geeigneten Base zum Auffangen der bei der Reaktion freiwerdenden Säure durchgeführt.

Als Lösungsmittel können verwendet werden aromatische Kohlenwasserstoffe wie Toluol oder Xylol, niedere Alkohole wie Methanol, Ethanol, Methylglycol oder 1-Butanol, Ether wie Tetrahydrofuran oder Glycoldimethylether, dipolar aprotische Lösungsmittel wie N,N-Dimethyformamid, N-Methylpyrrolidon, Acetonitril, Nitrobenzol, Dimethylsulfoxid oder Gemische dieser Lösungsmittel.

Geeignete Basen sind z. B. Alkali- oder Erdalkalimetallcarbonate oder -hydrogencarbonate wie Natriumhydrogencarbonat, Natriumcarbonat oder Calciumcarbonat, Alkali- oder Erdalkalimetallhydroxide wie Kaliumhydroxid oder

Bariumhydroxid, Alkoholate wie Natriumethanolat oder Kalium-tert.-butylat, lithiumorganische Verbindungen wie Butyllithium oder Lithiumdiisopropylamid, Alkali- oder Erdalkalimetallhydride wie Natriumhydrid oder Calciumhydrid, Alkalimetallfluoride wie Kaliumfluorid oder eine organische Base wie Triethylamin oder Pyridin.

Auch Zweiphasensysteme mit wäßrigen Lösungen von Basen in Gegenwart eines Phasentransferkatalysators wie z. B. Benzyltriethylammoniumchlorid, sind möglich.

In manchen Fällen ist der Zusatz eines Jodsalzes, z. B. Lithiumjodid, angebracht.

Die Reaktion wird gewöhnlich bei Temperaturen zwischen - 10 und 160 °C durchgeführt, vorzugsweise bei Raumtemperatur oder der Siedetemperatur des Lösungsmittels.

Die erfindungsgemäßen Arzneimittel können enteral (oral), parenteral (intravenös), rektal, subcutan, intramuskulär oder lokal (topisch) angewendet werden.

Sie können in Form von Lösungen, Pulvern (Tabletten, Kapseln einschließlich Microkapseln), Salben (Cremes oder Gele) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Als zweckmäßige Dosierung werden 0.1 - 10, vorzugsweise 0.2 - 8 mg/kg Körpergewicht ein- oder mehrmals täglich verabreicht. Die verwendeten

Dosierungseinheiten richten sich zweckmäßigerweise nach der jeweiligen Pharmakokinetik der verwendeten Substanz bzw. der verwendeten galenischen Zubereitung.

Die verwendete Dosierungseinheit der erfindungsgemäßen Verbindungen beträgt z. B. 1 - 1500 mg, vorzugsweise 50 - 500 mg.

Die erfindungsgemäßen Verbindungen können auch in Kombination mit anderen antiviralen Mitteln, wie z. B. Nucleosidanaloga, Proteaseinhibitoren oder Adsorptionsinhibitoren und Immunstimulantien, Interferonen, Interleukinen und koloniestimulierenden Faktoren (z. B. GM-CSF, G-CSF, M-CSF) verabreicht werden.

Wirksamkeitstests

Prüfung von Präparaten gegen HIV in der Zellkultur Methodenbeschreibung

Medium:
RPMI pH 6.8
Komplettes Medium enthält zusätzlich 20 % foetales Kälberserum und 40 IU/ml rekombinantes Interleukin 2.

Zellen:
Aus frischem Spenderblut mittels Ficol$^R$-Gradienten-Zentrifugation isolierte Lymphozyten werden unter Zusatz von 2 g/ml Phytohämagglutinin (Wellcome) in komplettem Medium 36 h bei 37°C unter 5 % C02 kultiviert. Die Zellen werden nach Zusatz von 10 % DMSO bei einer Zelldichte von 5 x $10^6$ eingefroren und in flüssigem Stickstoff gelagert. Für den Versuch werden die Zellen aufgetaut, im RPMI-Medium gewaschen und im kompletten Medium 3 - 4 Tage kultiviert.

Ansatz:
Die Prüfpräparate wurden in einer Konzentration von 16.7 mg/ml in DMSO gelöst und in komplettem Medium auf

1 mg/ml verdünnt.
In 24er Multiwell-Schalen wurden 0.4 ml Medium vorgelegt. Nach Zugabe von 0.1 ml des gelösten Präparates in die obere Reihe der Schale wurde durch Übertragung von jeweils 0.1 ml eine geometrische Verdünnungsreihe erzeugt. Präparatfreie Kontrollen enthielten stets 0.4 ml komplettes Medium mit 0.5% DMSO.
Lymphozytenkulturen mit einer Zellzahl von $5 \times 10^5$ Zellen/ml wurden durch Zugabe 1/50 Volumen Überstand aus HIV-infizierten Lymphozytenkulturen infiziert. Der Titer dieser Kulturüberstände wurde durch Endpunktverdünnung mit 1 - $5 \times 10^6$ infektiöse Einheiten/ml bestimmt. Nach 30 min Inkubation bei 37°C wurden die infizierten Lymphozyten abzentrifugiert und im gleichen Volumen Medium wieder aufgenommen. Von dieser Zellsuspension wurden jeweils 0.6 ml in alle Vertiefungen der Testplatte gegeben. Die Ansätze wurden 3 Tage bei 37°C inkubiert.

Auswertung:
Die infizierten Zellkulturen wurden unter dem Mikroskop auf Anwesenheit von Riesenzellen untersucht, die eine aktive Virusvermehrung in der Kultur anzeigen. Die geringste Präparatekonzentration, bei der keine Riesenzellen auftraten, wurde als Hemmkonzentration gegen HIV bestimmt. Zur Kontrolle wurden die Überstände aus den Kulturplatten mit Hilfe eines HIV-Antigentests entsprechend den Angaben des Herstellers (Organon) auf Anwesenheit von HIV-Antigen bestimmt.

Ergebnisse:
Die Ergebnisse dieses Tests zeigt Tabelle 1.

Tabelle 1

| Verbindungsnummer. | MHK (ng/ml) | EC-50 (ng/mt) |
|---|---|---|
| 5 | 200 | 5 |
| 15 | >40 | ≈ 20 |
| 16 | >40 | 50 |
| 18 | > 200 | ≈ 80 |
| 19 | ≈ 200 | ≈ 20 |
| 20 | > 8 | ≈ 2 |
| 30 | ≈ 200 | ≈ 40 |
| 34 | > 200 | ≈ 100 |
| 35 | > 40 | > 8 |
| 39 | > 200 | 30 |
| 40 | > 40 | 15 |
| 41 | > 40 | < 1 |
| 42 | > 200 | ≈ 100 |
| 45 | < 200 | ≈ 40 |
| 61 | ≈ 80 | n.d. |
| 70 | ≈ 400 | < 80 |
| 71 | ≈ 400 | <80 |
| 101 | ≈ 2000 | ≈ 400 |

Untersuchung der Substanzen auf Hemmung der HIV-"Reverse Transkriptase" Die Aktivität der Reversen Transkriptase (RT) wurde mit Hilfe eines "Scintillation Proximity Assay" (SPA) bestimmt.
Das Reagenzkit für den RT-SPA wurde von Amersham/Buchler (Braunschweig) bezogen. Das Enzym RT (aus HIV in E. coli cloniert) stammte von der Firma HT-Biotechnology LTD, Cambridge, UK.
Ansatz:
Der Test wurde nach dem Methoden-Manual des Herstellers Amersham durchgeführt - mit folgenden Modifikationen:

- dem "Assay"-Puffer wurde Rinderserumalbumin zu der Endkonzentration 0.5 mg/ml zugesetzt.

- der Test wurde in Eppendorf-Reaktionsgefäßen mit 100 ml Ansatzvolumen durchgeführt.
- das RT-Konzentrat des Herstellers (5000 U/ml) wurde in Tris-HCI Puffer 20 mM; pH 7.2; 30 % Glycerin auf eine Aktivität von 15 U/ml verdünnt.
- die Inkubationszeit für die Ansätze betrug 60 min (37°C).
- nach Abstoppen der Reaktion und "Entwicklung" mit der Perlen-Suspension wurden 130 ml Ansatz in 4.5 ml Tris-HCI Puffer, 10 mM; pH 7.4; 0.15 M NaCl transferiert und die Tritium-Aktivität in einem β-Counter gemessen.

Substanzprüfung:
Für eine Vorprüfung der Inhibitoraktivität wurden die Substanzen in DMSO gelöst (Stammlösung c = 1 mg/ml) und in Verdünnung in DMSO $10^{-1}$, $10^{-2}$, $10^{-3}$ usw. getestet.
Zur Bestimmung von $IC_{50}$-Werten wurden die Inhibitor-Stammlösungen in Tris-HCI Puffer, 50 mM, pH 8 weiterverdünnt und in geeigneten Konzentrationen getestet.
Aus der graphischen Darstellung RT-Aktivität versus Log $C_{Inh}$. wurde die einer 50 %igen Enzymhemmung zugehörige Konzentration ermittelt.
Die Ergebnisse der Untersuchung zeigt Tabelle 2.

Tabelle 2

| Verbindungsnummer | Reverse Transcriptase Assay IC-50 |
|---|---|
| 5 | 0,035 µM/ml |
| 6 | 0,022 µM/ml |
| 15 | 0,021 µM/ml |
| 16 | 0,372 µM/ml |
| 18 | 0,029 µM/ml |
| 19 | 0,094 µM/ml |
| 20 | 0,008 µM/ml |
| 30 | 0,147µM/ml |
| 34 | 0,415 µg/ml |
| 35 | 0,153 µg/ml |
| 40 | 0,170 µg/ml |
| 31 | 0,007 µM/ml |
| 43 | 0,026 µM/ml |
| 45 | 0,034 µM/ml |
| 61 | 0,026 µM/ml |
| 67 | 0,001 µM/ml |
| 70 | 0,008 µM/ml |
| 71 | 0,001 µM/ml |
| 101 | 0,041 µg/ml |

Durch die nachfolgenden Beispiele sowie durch den Inhalt der Patentansprüche wird die vorliegende Erfindung näher erläutert.
Herstellung der Ausgangsmaterialien

Beispiel I

Synthese von 5-Chlor-1-propionyl-isatin

18,2 g (0.1 mol) 5-Chlorisatin (Fa. Lancaster) werden in 10 ml Propionsäureanhydrid suspendiert und unter Rühren 2,5 Stunden unter Rückfluß erhitzt. Zur Aufarbeitung wird von der Reaktionsmischung das überschüssige Propionsäureanhydrid unter reduziertem Druck abdestilliert und der verbleibende Rückstand nach Abkühlung auf Zimmertemperatur mit 200 ml Diethylether versetzt. Der ausgefallene Feststoff wird abfiltriert und im Vakuum getrocknet.
Man erhält 14,9 g 5-Chlor-1-propionyl-isatin; rote Kristalle vom Schmelzpunkt 160 -162°C (Ausbeute: 63 % der Theorie)

Beispiel II

Synthese von 5-Chlor-3-(1-carboxy-prop-1-ylen)-indolin-2-on

Zu einer Lösung von 2,1 g (90 mmol) Natrium in 70 ml absolutem Ethanol werden bei Zimmertemperatur 5g (30 mmol) 5-Chlor-indolin-2-on, gelöst in 40 ml abs. Ethanol, gegeben. Nach beendeter Zugabe wird das Reaktionsgemisch mit 3,06 g (30 mmol) 2-Oxobuttersäure in 40 ml abs. Ethanol versetzt und 3 Stunden unter Rückfluß erhitzt. Das Reaktionsprodukt kristallisiert beim Abkühlen aus und wird von der Reaktionslösung abfiltriert, anschließend in 50 ml Wasser suspendiert, mit 2n wäßriger HCl acidifiziert und von der Lösung abgetrennt.
Man erhält 3,6 g gelbe Kristalle vom Schmelzpunkt 224 - 226°C, Ausbeute 48 % der Theorie

Beispiel III

Herstellung vom 5-Methoxyisatin

Stufe 1: Synthese von p-Methoxy-isonitrosoacetanilid

90 g Chloralhydrat werden in 1200 ml Wasser suspendiert und unter Rühren mit 1300 g Natriumsulfatdecahydrat versetzt. Diese Reaktionsmischung wird nacheinander mit 61,5 g p-Methoxyanilin (0,5 mol), 300 ml Wasser, 44 ml (0,52 mol) konz. Salzsäure und mit 110 g ( 1,58 mol) Hydroxyaminhydrochlorid, gelöst in 500 ml Wasser, versetzt. Die Reaktionslösung wird 5 min. unter Rückfluß erhitzt. Anschließend läßt man auf Raumtemperatur abgekühlen, wobei das Reaktionsprodukt auskristallisiert. Das ausgefallene Acetanilid wird abfiltriert, mit wenig Wasser gewaschen und im Vakuum getrocknet.
Man erhält 84,2 g hellbraune Kristalle vom Schmelzpunkt 182°C, Ausbeute: 87 % der Theorie.
$^1$H-NMR (200 MHz, $d_6$-DMSO): $\delta$ = 3,75 (s, 3H), 6,89 (m, 2 H), 7,60 (m, 2 H), 7,68 (s, 1 H), 10,1 (br s, 1H), 12,05 (s, 1H)

$$MS: (M + H)^+ = 195$$

Stufe 2: Synthese des Endproduktes

69 g der in Stufe 1 hergestellten Verbindung werden in 550 ml 90 % wäßriger Schwefelsäure gelöst, wobei die Temperatur der Lösung von Zimmertemperatur auf 55°C steigt. Es wird anschließend 30 Minuten bei dieser Temperatur gerührt und die Lösung dann auf 5 l Eiswasser gegeben. Der ausgefallene Reaktionsprodukt wird abfiltriert, gewaschen und im Ölpumpen-Vakuum getrocknet.
Man erhält 40 g farblose Kristalle vom Schmelzpunkt 190°C, Ausbeute 64 % der Theorie.
$^1$H-NMR (200 MHz, $d_6$-DMSO): $\delta$ = 3,76 (s, 3 H), 6,85 (d, J = 8,5 Hz, 1H), 7,06 (d, J = 2 Hz, 1 H), 7,20 (dd, 1 H), 10,82 (s, 1 N-H)

$$MS: (M + H)^+ = 178$$

Herstellung der Endprodukte

Beispiel 1

Herstellung von 6-Chlor-3-methyl-chinolin-2(1H)-on-4-carbonsäure (VI.**13** in Tabelle 4)

10,9 g (45,9 mmol) der in Beispiel I hergestellten Verbindung werden in 150 ml Wasser suspendiert und unter Rühren mit 2,1 Äquivalenten NaOH (3,9 g) versetzt. Anschließend wird das Reaktionsgemisch 1 Stunde unter Rückfluß erhitzt. Zur Aufarbeitung wird die noch heiße Lösung mit 1 g Aktivkohle versetzt und über Celite (Fa. Aldrich) filtriert.. Das auf diese Weise erhaltene Filtrat wird unter Eiskühlung mittels konz. Salzsäure angesäuert. Nach Entfernen des Lösungsmittels unter reduziertem Druck erhält man das Reaktionsprodukt als lachsfarbener Feststoff .
Man erhält: 8,1 g 6-Chlor-3-methyl-chinolin-2(1H)-on-4-carbonsäure vom Schmelzpunkt > 300°C; Ausbeute:

74 % der Theorie
$^1$H-NMR (200 MHz, $d_6$-DMSO); δ = 2,09 (s, 3 H), 7,3 - 7,7 (3 m, 3 H), 11,15 (br s, 1 N-H), 12,15 (br s, 1 N-H), 13,4 - 14,6 (br s, 1 COOH)

$$MS: (M + H)^+ = 238$$

Beispiel 2

Herstellung von 6-Chlor-3-methyl-chinolin-2(1H)-on-4-carbonsäurechlorid

6,5 g (0,027 mol) der in Beispiel 1 hergestellten Carbonsäure werden in 80 ml Thionylchlorid gelöst und zwei Stunden unter Rückfluß erhitzt.
Zur Aufarbeitung wird das Reaktionsgemisch am Rotationsverdampfer unter reduziertem Druck eingeengt unter der verbleibende Rückstand mit Diethylether verrührt. Das Reaktionsprodukt wird abfiltriert und der erhaltene Feststoff im Vakuum getrocknet.
Man erhält 5,6 g rötlich gefärbte Kristalle vom Schmelzpunkt 179°C (81% der Theorie)
Das auf diese Weise erhaltene Reaktionsprodukt wird ohne weitere Reinigung für weitere Reaktionen verwendet (siehe Beispiel 3).

Beispiel 3

Herstellung von 6-Chlor-4-hydroxymethyl-3-methyl-chinolin-2(1H)-on

1,28 g (5 mmol) des in Beispiel 2 hergestellten Säurechlorids werden in 50 ml absolutem Tetrahydrofuran (THF) gelöst und unter Rühren mit 0,38 g (10 mmol) Natriumborhydrid und 0,5 ml Wasser versetzt. Anschließend wird 1 Stunde unter Rückfluß erhitzt.
Zur Aufarbeitung wird die Reaktionslösung am Rotationsverdampfer unter reduziertem Druck eingeengt, der Rückstand in 100 ml Wasser aufgenommen und mit 20 % wäßriger Zitronensäurelösung angesäuert. Der ausgefallene Feststoff wird abfiltriert und im Vakuum getrocknet.
Man erhält 0,720 g farblose Kristalle vom Schmelzpunkt 330°C (Produkt schmilzt unter Zersetzung); Ausbeute 64 %.
$^1$H-NMR (200 MHz, $d_6$-DMSO): δ = 2,18 ( s, 3 H), 4,71 ( d, J = 9 Hz, 2 ), 5.32 (t, J = 9 Hz, 1 OH), 7,28 ( d, J = 8 Hz), 7,50 (dd, J = 2 Hz), 7,89 (d, 1H), 11,85 (br s, 1 N-H)

$$MS: (M + H)^+ = 224$$

Beispiel 4

Herstellung von 4-Acetoxymethyl-6-chlor-3-methyl-chinolin-2(1H)-on und 4-Acetoxymethyl-1-acetyl-6-chlor-3-methyl-chinolin-2(1H)-on

700 mg (3,13 mmol der nach Beispiel 3 hergestellten Hydroxymethyl-Verbindung werden in 50 ml Essigsäureanhydrid gelöst und mit 3 g Natriumacetat versetzt. Das Reaktlonsgemisch wird 2 Stunden auf 100°C erhitzt. Zur Aufarbeitung wird am Rotationsverdampfer unter reduziertem Druck eingeengt und der verbleibende Rückstand mit Methytenchlorid/Wasser extrahiert. Die organische Phase wird mittels Natriumsulfat getrocknet und das Lösungsmittel am Rotationsverdampfer unter reduziertem Druck entfernt. Das auf diese Weise erhaltene Rohprodukt besteht aus 2 Reaktionsprodukten, welche mittels Chromatographie an Kieselgel (mobile Phase: n-Heptan/Essigsäureethylester = 2/1) isoliert werden.
Nach chromatographischer Reinigung und Entfernen des verwendeten Elutionsmittels am Rotationsverdampfer erhält man beide Reaktionsprodukte nach Verrühren der jeweiligen Fraktionen mit Diethylether in kristalliner Form. Bei den beiden Verbindungen handelt es sich um 4-Acetoxymethyl-6-chlor-3-methyl-chinolin-2(1H)-on und 4-Acetoxymethyl-1-acetyl-6-chlor-3-methylchinolin-2(1H)-on handelt.

4-Acetoxymethyl-6-chlor-3-methyl-chinolin-2(1H)-on Ausbeute: 480 mg, farblose Kristalle vom Schmelzpunkt 220°C; $R_F$ (n-Heptan/Essigsäureethylester = 2:1) = 0,08

$^1$H-NMR (200 MHz, $d_6$-DMSO): = 2,06 (s, 3 H), 2,21 (s, 3 H), 5,39 (ps s, 2 H), 7,32 (s, J = 8 Hz, 1 H), 7,51 (dd, J = 2 Hz, 1H), 7,81 (d, 1 H)

$$MS: (M + H)^+ = 266$$

4-Acetoxymethyl-1-acetyl-6-chlor-3-methyl-chinolin-2(1H)-on Ausbeute: 270 mg, farblose Kristalle vom Schmelzpunkt 116 °C; $R_F$ (n-Heptan/Essigsäureethylester = 2:1) = 0,23

$^1$H-NMR (200 MHz, $d_6$-DMSO): δ = 2,04 (s, 3 H), 2,38 (s, 3H), 2,42 (s, 3 H), 5,59 (m, 2H), 7,79 (dd, J =9 und 2 Hz, 1 H), 7,95 (d, 1 H), 8,26 (d, 1H)

$$MS: (M + H)^+ = 308$$

Beispiel 5

Synthese von 4-Acetoxymethyl-6-chlor-3-methyl-chinolin-2(1H)-thion

266 mg (1 mmol) 4-Acetoxymethyl-6-chlor-3-methyl-chinolin-2(1H)-on (siehe Beispiel 4) werden in 40 ml absolutem Toluol suspendiert und mit 222 mg (0,55 mmol) Lawesson's Reagenz (2,4-Bis-(4-methoxyphenyl)-1,3-dithia-2,4-diphosphetan-2,4-disulfid) versetzt und 1 Stunde auf 100°C erhitzt. Zur Aufarbeitung wird das Reaktionsgemisch am Rotationsverdampfer unter reduziertem Druck eingeengt und das Rohprodukt mittels Chromatographie an Kieselgel (mobile Phase: n-Heptan/Essigsäureethylester = 4/1) gereinigt. Man erhält ein gelbes Öl, das nach Zugabe von n-Pentan in kristalliner Form anfällt.

Ausbeute: 90 mg, gelbe Kristalle vom Schmelzpunkt 235°C, $R_F$ (n-Heptan/Essigsäureethylester = 2:1) = 0,46
$^1$H-NMR (200 MHz, $d_6$-DMSO): δ = 2,03 (s, 3H), 3,29 (s, 3 H), 5,45 (ps s, 2 H), 7,60 - 7,75 (m, 2 H), 7,97 (ps s, 1 H), 13,95 (br s, 1 N-H)

$$MS: (M + H)^+ = 282$$

Beispiel 6

Herstellung von 3-Phenyl-chinolin-2(1H)-on-4-carbonsäure (VI.1 in Tabelle 4)

25 g Isatin (0,17 mol) werden mit 50,9 g (0,374 mol) Phenylessigsäure und 2,5 g wasserfreiem Natriumacetat unter Rühren 30 min. auf 220°C erhitzt. Nach Zusatz von 150 ml Eisessig bei dieser Temperatur, läßt man Abkühlen und versetzt das Reaktionsgemisch mit Eiswasser. Ausgefallenes Reaktionsprodukt wird abfiltriert und im Vakuum getrocknet. Das Rohprodukt wird aus Eisessig unter Verwendung von Aktivkohle umkristallisiert.

Man erhält: 21,3g Phenyl-chinolin-2(1H)-on-4-carbonsäure vom Schmelzpunkt 288°C in Form hellbeiger Kristalle; Ausbeute: 74 % der Theorie
$^1$H-NMR (200 MHz, $d_6$-DMSO): δ = 7,20 - 7,65 (m, 9 H), 12,16 (br s, 1 N-H), 13,8 - 14,0 (br s, 1-COOH)

$$MS: (M + H)^+ = 266$$

Beispiel 7

Herstellung von 3-Phenyl-chinolin-2(1H)-on-4-carbonsäure-isopropylester

1,42 g (5 mmol) der aus Verbindung 6 mittels des in Beispiel 2 beschriebenen Verfahrens hergestellten Säurechlorids löst man in 25 ml absolutem Isopropanol, welches vorher mit 0,290 g (12,5 mmol) Natrium-Metall versetzt worden war. Anschließend erhitzt man das Reaktionsgemisch 4 Stunden auf Rückflußtemperatur. Nach Beendigung der Reaktion wird das Reaktionsgemisch unter reduziertem Druck am Rotationsverdampfer eingeengt, der verbleibende Rückstand mit Wasser verrührt und der ausgefallene Feststoff abgesaugt. Zur weiteren Reinigung des Reaktionsproduktes wird aus Isopropanol unter Verwendung von Aktivkohle umkristallisiert.

Man erhält 0,8 g leicht rosa-farbene Kristalle vom Schmelzpunkt 211 - 212°C; $R_F$-Wert = 0,57 (Laufmittel: Essigsäureethylester/n-Heptan = 2/1); Ausbeute 52 % der Theorie

$^1$H-NMR (200 MHz, $d_6$-DMSO): $\delta$ = 0,98 (d, J = 7 Hz, 6 H), 4,97 (hept, 1 H), 7,20 - 7,65 (m, 9 H), 12,22 (br s, 1 N-H)

MS: $(M + H)^+$ = 308

Beispiel 8

Herstellung von 3-Phenyl-chinolin-2(1H)-thion-4-carbonsäure-isopropylester

307 mg (1 mmol) 3-Phenyl-chinolin-2(1H)-on-4-carbonsäure-isopropylsäureester (Beispiel 7) werden nach dem in Beispiel 5 beschriebenen Verfahren mit Lawesson's Reagenz umgesetzt und wie oben angegeben mittels Chromatographie an Kieselgel gereinigt (mobile Phase: n-Heptan/Essigsäureethylester = 4/1).

Man erhält 0,29 g gelbliche Kristalle vom Schmelzpunkt 235°C; Ausbeute 90 % der Theorie.

$^1$H-NMR (200 MHz, $d_6$-DMSO): $\delta$ = 0,95 (d, J = 7 Hz, 6 H), 4,91 (hept, 1 H), 7,18 - 7,24 (m, 2H), 7,35 - 7,55 (m, 5 H), 7.64 - 7,80 (m, 2 H), 14,02 ( br s, 1 N-H)

MS: $(M + H)^+$ = 324

Beispiel 9

Herstellung von 6-Chlor-3-ethyl-chinolin-2(1H)-on-4-carbonsäure (VI.14 in Tabelle 4)

3,4 g 5-Chlor-3-(1-carboxy-prop-1-ylen)-indolin-2-on (Beispiel II) werden in 270 ml 2 n HCl suspendiert und 20 Stunden unter Rückfluß erhitzt. Nach Beendigung der Reaktion läßt man auf Zimmertemperatur abkühlen und filtriert das auskristallisierte Reaktionsprodukt ab. Zur weiteren Reinigung der erhaltenen Carbonsäure wird diese nochmals mit einem Gemisch aus 100 ml Wasser und 100 ml Ethanol verrührt, abfiltriert und getrocknet.

Man erhält 1,45 g braune Kristalle vom Schmelzpunkt 215 - 217°C , Ausbeute: 42 % der Theorie.

$^1$H-NMR (200 MHz, $d_6$-DMSO): $\delta$ =1,13 (t, J = 7.5 Hz, 2 H), 2,52 (q, 2H), 7,15 - 7,6 (m, 3 H), 12,15 (br s, 1H, N-H), 13,0 - 14,6 (br s, 1 COOH)

MS: $(M + H)^+$ = 252

Beispiel 10

Herstellung von 6-Chlor-3-ethyl-chinolin-2(1H)-on-4-carbonsäureisopropylester

1,45 g (6 mmol) der aus Verbindung des Beispiels 9 mittels des in Beispiel 2 beschriebenen Verfahrens hergestellten Säurechlorids werden in 25 ml absolutem Isopropanol, welches vorher mit 0,173 g ( 7,5 mmol) Natrium-Metall versetzt worden war, gelöst und erhitzt das Reaktionsgemisch anschließend 3 Stunden unter Rückfluß. Nach Beendigung der Reaktion wird die erhaltene Suspension unter reduziertem Druck am Rotationsverdampfer eingeengt, der verbleibende Rückstand mit 200 ml Essigsäureethylester aufgenommen und die organische Phase mit Wasser gewaschen. Anschließend wird das Rohprodukt mittels Natriumsulfat getrocknet und das organische Lösungsmittel unter reduziertem Druck am Rotationsverdampfer entfernt Das resultierende gelbe Öl wird mittels Chromatographie an Kieselgel (mobile Phase: N-Heptan/Essigsäureethylester = 1/1) gereinigt.

Ausbeute: 0,280 g (32 % der Theorie), farblose Kristalle vom Schmelzpunkt 179 - 180°C

$^1$H-NMR (200 MHz, $d_6$-DMSO): $\delta$ = 1,09 (t, J = 7,5 Hz, 3 H), 1,38 (d, J = 7 Hz, 6 H), 2,49 (q, 2 H), 5,37 (hept, 1 H), 7,34 (d, J = 2 Hz), 7,36 (d, J = 8 Hz), 7.58 (dd, 1H), 12,18 (br s, 1 H)

MS: $(M + H)^+$ = 294

Beispiel 11

Herstellung von 6-Chlor-3-ethyl-chinolin-2(1H)-thion-4-carbonsäureisopropylester

150 mg (0,51 mmol) 6-Chlor-3-ethyl-chinolin-2(1H)-on-4-carbonsäureisopropylester (Beispiel 10) werden nach dem in Beispiel 5 beschriebenen Verfahren mit Lawesson's Reagenz umgesetzt (Reaktionszeit 5 Stunden bei Rückflußtemperatur) und wie oben angegeben mittels Chromatographie an Kieselgel gereinigt (mobile Phase: n-Heptan/Essigsäureethylester = 2/1).

Man erhält 0,13 g gelbliche Kristalle vom Schmelzpunkt 194 - 196°C; Ausbeute: 90 % der Theorie
$^1$H-NMR (200 MHz, $d_6$-DMSO): δ =1,18 (t, J = 7,5 Hz, 3 H), 1,40 (d, J = 7 Hz, 6 H), 3,86 (q, 2 H), 5,39 ( hept, 1 H), 7,45 ( ps s, 1 H), 7,65 - 7,80 (m, 2 H), 14,05 ( bs s, 1 N-H)

MS: $(M + H)^+$ = 310

Beispiel 12

Herstellung von 6-Methoxy-3-phenyl-chinolin-2(1H)-on-4-carbonsäure (VI.21 in Tabelle 4)

8,5 g 5-Methoxyisatin (50 mmol, Beispiel III) werden gemäß dem in Beispiel 6 beschriebenen Verfahren mit 15 g (50 mmol) Phenylessigsäure umgesetzt. Zur Aufarbeitung wird mit 600 ml Eiswasser versetzt und die Lösung nach Alkalisierung mittels konz. NaOH mit jeweils 100 ml Dichlormethan zweimal extrahiert. Die wäßrige Phase wird anschließend mit konz. Salzsäure angesäuert und das ausgefallene Reaktionsprodukt abfiltriert.

Man erhält 10,9 g grau gefärbte Kristalle, welche bei 320 °C unter Zersetzung schmelzen; Ausbeute: 74 % der Theorie
$^1$H-NMR (200 MHz, $d_6$-DMSO): δ = 3,79 (s, 3 H), 6,88 (m, 1 H), 7,20 - 7,48 (m, 7 H), 12,05 (br s, 1H), 13,8 (br s, 1 H)

MS: $(M + H)^+$ = 296

Beispiel 13

Herstellung von 6-Methoxy-3-phenyl-chinolin-2(1H)-on-4-carbonsäureisopropylester

3,1 g (10 mmol) der aus Verbindung des Beispiels 12 mittels des in Beispiel 2 beschriebenen Verfahrens hergestellten Säurechlorids löst man in 50 ml absolutem Isopropanol, welches man vorher mit 0,58 g ( 25 mmol) Natrium-Metall versetzt hatte, erhitzt das Reaktionsgemisch 4 Stunden auf 50°C und rührt dann 4 Stunden bei Zimmertemperatur. Nach Beendigung der Reaktion wird die erhaltene Suspension unter reduziertem Druck am Rotationsverdampfer eingeengt, der verbleibende Rückstand mit 100 ml 20 % wäßriger Zitronensäurelösung verrührt und das Reaktionsprodukt abgesaugt. Zur weiteren Reinigung des Rohproduktes wird aus absolutem Ethanol unter Verwendung von Aktivkohle umkristallisiert.

Man erhält 2 g rötlich gefärbte Kristalle vom Schmelzpunkt 231 °C; Ausbeute 60 % der Theorie
$^1$H-NMR (200 MHz, $d_6$-DMSO): δ = 1,02 (d,J = 7 Hz, 6 H), 3,78 (s, 3 H), 4,99 (hept, 1 H), 6,83 (d, J = 2 Hz, 1 H), 7,22 - 7,48 (m, 7 H), 12,15 (br s, 1 N-H)

MS: $(M + H)^+$ = 338

Beispiel 14

Herstellung von 6-Methoxy-3-phenyl-chinolin-2(1H)-thion-4-carbonsäureisopropylester

1 g (3 mmol) 6-Methoxy-3-phenyl-chinolin-2(1H)-on-4-carbonsäureisopropylester (Beispiel 13) werden gemäß Beispiel 5 mit 1,1 Äquivalenten Lawesson's Reagenz umgesetzt (Reaktionszeit 1 Stunde; Reaktionstemperatur: 100°C). Nach Einengen des Lösung wird das Reaktionsprodukt mittels Chromatographie an Kieselgel gereinigt (mobile Phase: n-Heptan/Essigsäureethylester = 1/1).

Man erhält 840 mg gelbe Kristalle vom Schmelzpunkt 219°C, Ausbeute 79 % der Theorie
$^1$H-NMR (200 MHz, $d_6$-DMSO): δ = 0,96 ( d, J = 7 Hz, 6 H), 3,80 (s, 3 H), 4,93 (hept, 1 H), 6,82 (d, J = 2 Hz, 1 H), 7,16-

7,24 (m, 2 H), 7,32 - 7,45 (m, 4 H), 7,72 (d, J = 9 Hz, 1 H), 14,02 (br s, 1 N-H)

MS: $(M + H)^+ = 354$

Beispiel 15

Herstellung von 6-Chlor-3-(p-chlorphenyl)-4-isopropoxycarbonyl-chinolin-2(1H)-on-2-O-methyloxim

0,42 g(1,07 mol) 6-Chlor-3-(p-chlorphenyl)-4-isopropoxycarbonyl-chinolin-2(1H)-thion (hergestellt nach Methoden der oben beschriebenen Beispiele 6, 7 und 8, jedoch unter Verwendung von 5-Chlorisatin und p-Chlorphenylessigsäure als Ausgangsmaterialien) werden in 10 ml abs. Ethanol gelöst und mit 0,5 g O-Methylhydroxylaminhydrochlorid versetzt. Das Reaktionsgemisch wird 3 Stunden auf Rückflußtemperatur erhitzt. Anschließend wird am Rotationsverdampfer unter reduziertem Druck eingeengt, der Rückstand in Ethylacetat aufgenommen, die organische Phase zweimal mit Wasser gewaschen und nach Trocknung der organischen Phase mittels Natriumsulfat am Rotationsverdampfer unter vermindertem Druck eingeengt. Das erhaltene Rohprodukt wird mittels Chromatographie an Kieselgel gereinigt (mobile Phase: Ethylacetat/n-Heptan = 1/4). Zur Feinreinigung ist eine Chromatographie an ®Sephadex Typ LH-20 mit Methanol als mobiler Phase geeignet.

Man erhält 80 mg gelbe Kristalle vom Schmelzpunkt 178 - 180°C, Ausbeute 18 % der Theorie.
$^1$H-NMR (200 MHz, $d_6$-DMSO): δ = 0,93 (d, J = 7 Hz, 6H), 3,68 (s, 3 H), 4,90 (hept, 1H), 7,1 (d, J = 2 Hz, 1 H), 7,22 - 7,34 (m, 2 H), 7,40 -7,56 (m, 4 H), 10,40 (br s, 1 N-H)

MS: $(M + H)^+ = 405$

Beispiel 16

Herstellung von 4-Benzoyl-6-chlor-3-phenyl-chinolin-2(1H)-on

1 g (3,1 mmol) 6-Chlor-3-phenyl-chinolin-2(1H)-on-4-carbonsäurechlorid (hergestellt in Analogie zu den in den Beispielen 6 und 2 beschriebenen Verfahren, jedoch unter Verwendung von 5-Chlorisatin und Phenylessigsäure als Ausgangskomponenten) werden in 30 ml abs. Tetrahydrofuran gelöst und auf -50°C gekühlt. Anschließend werden 2,1 ml (2 Moläquivalente) einer 3 molaren etherischen Lösung zugetropft. Nach beendeter Zugabe wird 30 min. bei -40°C und 2 Stunden bei 20°C gerührt.

Zur Aufarbeitung wird die Reaktionsmischung auf 20 %ige wässrige Zitronensäurelösung gegossen und diese Lösung zweimal mit 150 ml Essisäureethylester extrahiert. Die vereinigten organischen Phasen werden über $Na_2SO^-_4$ getrocknet und unter reduziertem Druck am Rotationsverdampfer eingeengt. Das Rohprodukt fällt als hellgelbes Öl an, welches mittels Chromatographie an Kieselgel (mobile Phase Ethylacetat/n-Heptan = 3/2) gereinigt wird.

Man erhält 140 mg leicht rosafarbene Kristalle vom Schemlzpunkt 266-267°C [$R_F$ (Ethylacetat/n-Heptan = 1/1) = 0,24], Ausbeute 13 % der Theorie.
$^1$H-NMR (200 MHz, $d_6$-DMSO): δ = 7,05 - 7,78 (m, 13H), 12,42 (br s, 1H).

MS: (M + H) = 360

Die folgende Tabelle 3 gibt eine Übersicht der synthetisierten Verbindungen. Alle Derivate wurden mittels $^1$H-NMR-Spektrum, Massenspektrum sowie über den jeweiligen Schmelzpunkt charakterisiert.

Tabelle 3: Synthetisierte Derivate der Formeln (I) und (Ia)

Die Herstellung der in Tabelle 3 aufgeführten Derivate der Formeln I und Ia erfolgt i.a. aus den betreffenden Carbonsäuren der Formeln VI oder VIa, welche sich nach in den Beispielen 1, 6, 9 und 12 beschriebenen Methoden synthetisieren lassen. Die Verbindungen der Formeln VI und VIa sind entweder literaturbekannt oder können in Analogie zu literaturbekannten Verfahren hergestellt werden (vergl. z. B.: J. Am. Chem. Soc. 1953, 75, 5305; Ind. J. Chem. 1987, 26B, 910; Synthesis 1985, 541; J. Heterocyclic Chem. 1979, 16, 487; J. Heterocyclic Chem. 1989, 26, 281; Ber. 1894, 26, 2484; Bull. Chem. Soc. Japan 1977, 50(8), 1959; J. Org. Chem. 1972, 37, 3967; Chem. Pharm.Bull. 1992, 40, 1322; des weiteren Lehrbücher der Organischen Chemie, z. B. "The Chemistry of Heterocyclic Compounds", E.C. Taylor (Ed.), Wiley & Sons, New York oder "Advances in Heterocyclic Chemistry", A.R. Katritzky

(Ed.), Academic Press, San Diego). Weiterhin sind die als Ausgangsmaterialien verwendeten Isatin- bzw. Idolin-2-on-Derivate entweder käuflich oder können nach bekannten, in der Literatur beschriebenen Verfahren synthetisiert werden (vergl. z.B. J. Org. Chem. 1977, 42, 1344; J. Prak. Chem. 1940, 155, 234; Org. Syntheses, Coll. Vol. 1, 327 (1941); Ind. J. Chem 1990, 29B, 578; Pharmazie 1984, 39, 153; Ind. J. Chem 1987, 26B, 535; J. Am. Chem. Soc. 1974, 96, 5512; J. Org. Chem. 1979, 44, 628; J. Med. Chem. 1992, 35, 2085;des weiteren Lehrbücher der Organischen Chemie, z. B. "The Chemistry of Heterocyclic Compounds", E.C. Taylor (Ed.), Wiley & Sons, New York oder

| Verbindungsnummer | Struktur | Schmelzpunkt (in °C) | Herstellung gem. Beisp.: Ausbeute in % | eingesetztes Edukt |
|---|---|---|---|---|
| 1 | 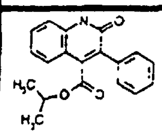 | 212 | siehe Beispiel 7, 52 % | VI.1 |
| 2 | 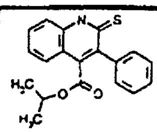 | 235 | siehe Beispiel 8, 90 % | 1 |
| 3 | 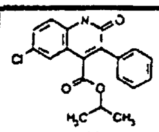 | 246 | analog Beispiel 7, 63 | VI.2 |
| 4 | 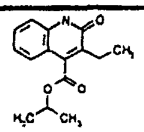 | 124-126 | analog Beispiel 7, 58 % | VI.3 |
| 5 | 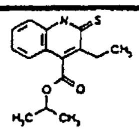 | 154 - 155 | analog Beispiel 5, 82 % | 4 |
| 6 | 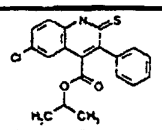 | - 234 | analog Beispiel 5, 78 % | 3 |
| 7 | 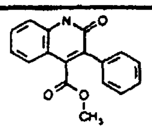 | 248 - 252 | analog Beispiel 7, 84 % | VI.1 |
| 8 | 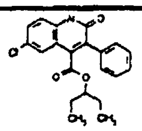 | 190 - 193 | analog Beispiel 7, 27 % | VI.2 |
| 9 | 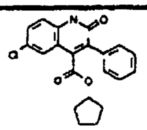 | 211 - 214 | analog Beispiel 7, 68 % | VI.2 |
| 10 | 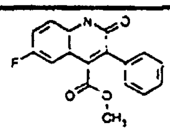 | 287 - 289 | analog Beispiel 7, 96 % | VI.5 |
| 11 | 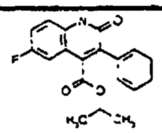 | 233 - 236 | analog Beispiel 7 90 % | VI.5 |
| 12 | 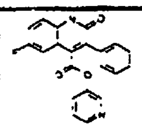 | 254 - 257 | analog Beispiel 7, 78 % | VI.5 |

| | | | | |
|---|---|---|---|---|
| 13 | 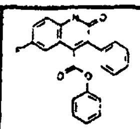 | 263 - 265 | analog Beispiel 7, 71 % | VI.5 |
| 14 | 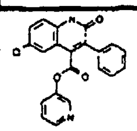 | 253 - 255 | analog Beispiel 7, 62 % | VI.2 |
| 15 | 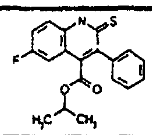 | 210 - 213 | analog Beispiel 5, 26 % | 11 |
| 16 | 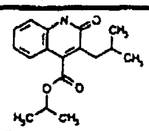 | 162 -164 | analog Beispiel 7, 35 % | VI.4 |
| 17 | 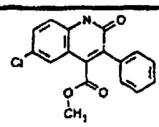 | 273 - 275 | analog Beispiel 7, 80 % | VI.2 |
| 18 | 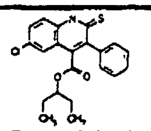 | 185 - 186 | analog Beispiel 5, 41 % | 8 |
| 19 | 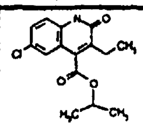 | 179 - 180 | siehe Beispiel 10 | VI.14 |
| 20 | 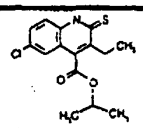 | 193 - 195 | siehe Beispiel 11 | 19 |
| 21 | 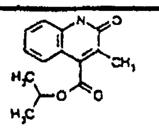 | 185 | analog Beispiel 7, 35 % | VI.10 |
| 22 | 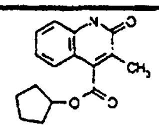 | 183 | analog Beispiel 7, 44 %. | VI.10 |
| 23 | 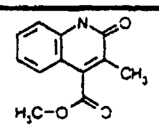 | 174 | analog Beispiel 7, 68 % | VI.10 |
| 24 | 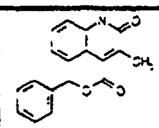 | 189 | analog Beispiel 7, 59 % | VI.10 |
| 25 | 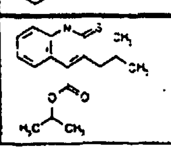 | 179 -180 | analog Beispiel 5, 83 % | 16 |

| | | | | |
|---|---|---|---|---|
| 26 | 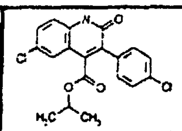 | 210 215 | analog Beispiel 7, 67 % | VI.15 |
| 27 | 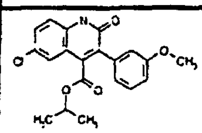 | 268 - 270 | analog Beispiel 7, 85 % | VI.17 |
| 28 | 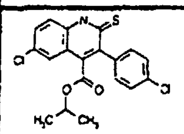 | 220 - 223 | analog Beispiel 5, 60 % | 26 |
| 29 | 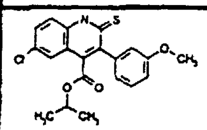 | 216 -218 | analog Beispiel 5, 14 % | 27 |
| 30 | 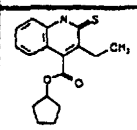 | 169 - 170 | analog Beispiel 5, 71 % | 98 |
| 31 | 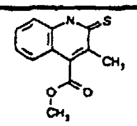 | 217 | analog Beispiel 5, 59 % | 23 |
| 32 | 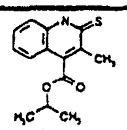 | 195 | analog Beispiel 5, 86 % | 21 |
| 33 | 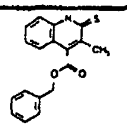 | 192 | analog Beispiel 5, 74 % | 24 |
| 34 | 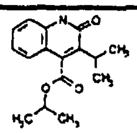 | 140 | analog Beispiel 7, 61 % | V.11 |
| 35 | 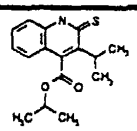 | 160 | analog Beispiel 5, 95 % | 34 |
| 36 | 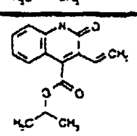 | 158 | analog Beispiel 7, 34 % | VI.6 |
| 37 | 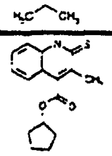 | 191 | analog Beispiel 5, 92 % | 23 |
| 38 | 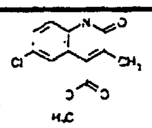 | 253 | analog Beispiel 7, 62 % | VI.13 |

| | | | | |
|---|---|---|---|---|
| 39 | 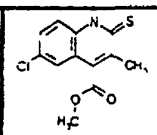 | 237 | analog Beispiel 5, 90 % | 38 |
| 40 | 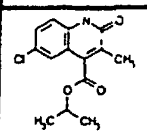 | 213 | analog Beispiel 7, 46 % | VI.13 |
| 41 | 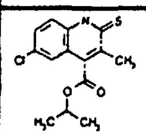 | 209 | analog Beispiel 5, 94 % | 40 |
| 42 | 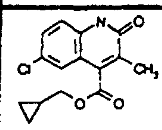 | 202 | analog Beispiel 7, 59 % | VI.13 |
| 43 | 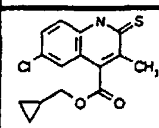 | 187 | analog Beispiel 5, 90 % | 42 |
| 44 | 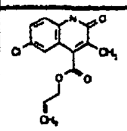 | 214 | analog Beispiel 7, 64 % | VI.13 |
| 45 | 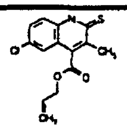 | 216 | analog Beispiel 5, 97 % | 44 |
| 46 | 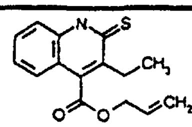 | 178 - 179 | analog Beispiel 5, 59 % | 50 |
| 47 | 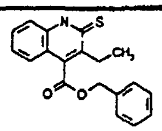 | 176 -178 | analog Beispiel 5, 79 % | 49 |
| 48 | 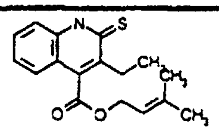 | 121 -123 | analog Beispiel 5, 5 % | 51 |
| 49 | 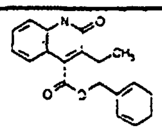 | 174 | analog Beispiel 7, 64 % | VI.3 |
| 50 | 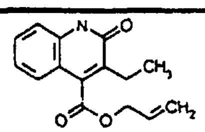 | 153 -154 | analog Beispiel 7, 95 % | VI.3 |
| 51 | 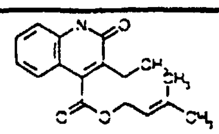 | 101 -102 | analog Beispiel 7, 95 % | VI.3 |

| | | | | |
|---|---|---|---|---|
| 52 | 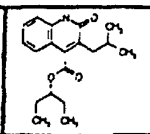 | 146 - 147 | analog Beispiel 7, 48 % | VI.4 |
| 53 | 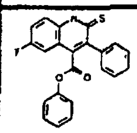 | 238 -242 | analog Beispiel 5, 56 % | 13 |
| 54 | 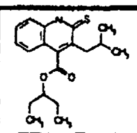 | 157 - 158 | analog Beispiel 5, 55 % | 52 |
| 55 | 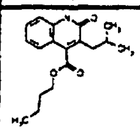 | 119 - 120 | analog Beispiel 7, 82 % | VI.4 |
| 56 | 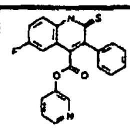 | 250 - 252 | analog Beispiel 5, 68 % | 12 |
| 57 | 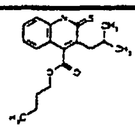 | 129 - 130 | analog Beispiel 5, 82 % | 55 |
| 58 | 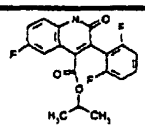 | 245 - 250 | analog Beispiel 7, 36 % | VI.7 |
| 59 | 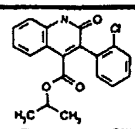 | 212 - 215 | analog Beispiel 7, 88 % | VI.12 |
| 60 | 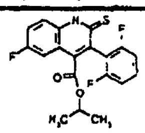 | 246 -248 | analog Beispiel 5, 58 % | 58 |
| 61 | 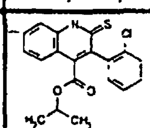 | 264 -266 | analog Beispiel 5, 49 % | 59 |
| 62 | 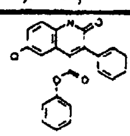 | 272 -275 | analog Beispiel 7, 61 % | VI.2 |
| 63 | 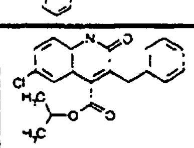 | 226 | analog Beispiel 7, 28 % | VI.8 |
| 64 | 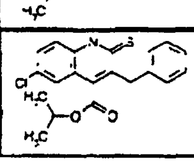 | 200 | analog Beispiel 5, 42 % | 63 |

| | | | | |
|---|---|---|---|---|
| 65 | 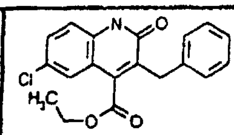 | 200 | analog Beispiel 7, 54 % | VI.8 |
| 66 | 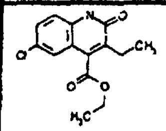 | 175 - 175 | analog Beispiel 7, 27 % | VI.14 |
| 67 | 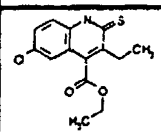 | 184 - 185 | analog Beispiel 5, 82 % | 66 |
| 68 | 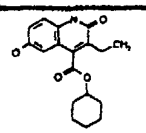 | 223 | analog Beispiel 7, 5 % | VI.14 |
| 69 | 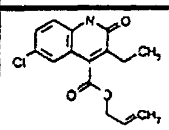 | 174 - 175 | analog Beispiel 7, 27 % | VI.14 |
| 70 | 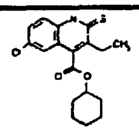 | 184 | analog Beispiel 5, 94 % | 68 |
| 71 | 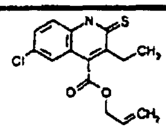 | 187 -189 | analog Beispiel 5, 79 % | 69 |
| 72 | 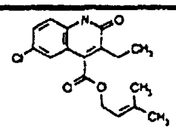 | 141 -142 | analog Beispiel 7, 21 % | VI.14 |
| 73 | 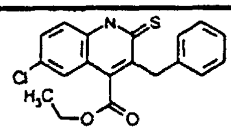 | 209 | analog Beispiel 5, 56 % | VI.8 |
| 74 | 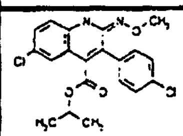 | 178 - 180 | siehe Beispiel 15, 19 % | 28 |
| 75 | 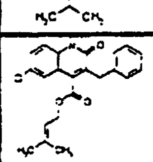 | 202 | analog Beispiel 7, 35 % | VI.8 |
| 76 | 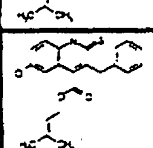 | 160 | analog Beispiel 5, 35 % | 75 |
| 77 | 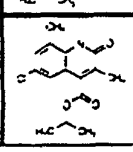 | 214 | analog Beispiel 7, 56 % | VI.22 |

| | | | | |
|---|---|---|---|---|
| 78 | 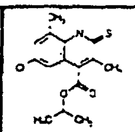 | 187 | analog Beispiel 5, 95 % | 77 |
| 79 | 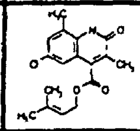 | 179 | analog Beispiel 7, 70 % | VI.22 |
| 80 | 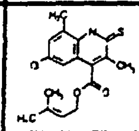 | 125 | analog Beispiel 5, 83 % | 79 |
| 81 | 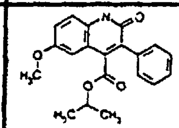 | 231 | siehe Beispiel 13, 60 % | VI.21 |
| 82 | 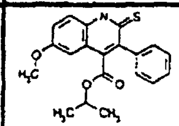 | 219 | siehe Beispiel 14, 79 % | 81 |
| 83 | 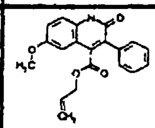 | 229 | analog Beispiel 7, 72 % | VI.21 |
| 84 | 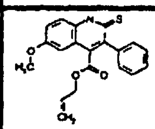 | 224 | analog Beispiel 5, 53 % | 83 |
| 85 | 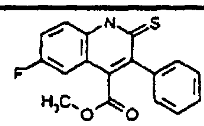 | 227 - 228 | analog Beispiel 5, 15 % | 10 |
| 86 | 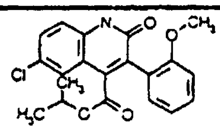 | 220 - 223 | analog Beispiel 7, 55 % | VI.16 |
| 87 | 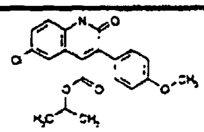 | 230 -232 | analog Beispiel 7, 64 % | VI.18 |
| 88 | 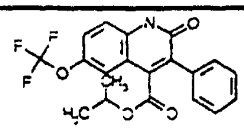 | 194 - 195 | analog Beispiel 7, 78 % | VI.9 |
| 89 | 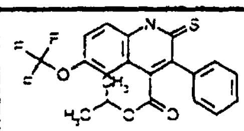 | 210 - 212 | analog Beispiel 5, 65 % | 88 |
| 90 | 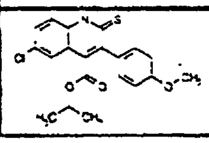 | 228 - 230 | analog Beispiel 5, 63 % | 87 |

| | | | | |
|---|---|---|---|---|
| 91 | 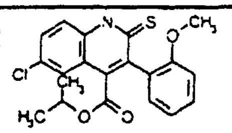 | 251 - 254 | analog Beispiel 5, 40 % | 86 |
| 92 | 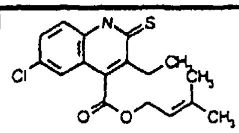 | 154 | analog Beispiel 5, 57 % | 72 |
| 93 | 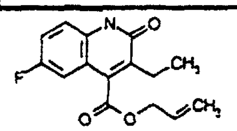 | 148 | analog Beispiel 7, 58 % | VI.19 |
| 94 | 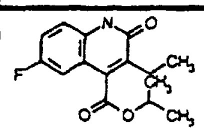 | 144 | analog Beispiel 7, 61 % | VI.19 |
| 95 | 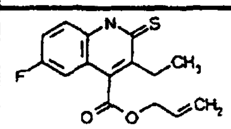 | 183 | analog Beispiel 5, 82 % | 93 |
| 96 | 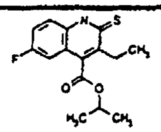 | 184 | analog Beispiel 5, 98 % | 94 |
| 97 | 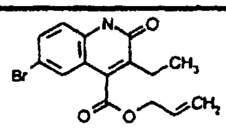 | 187 | analog Beispiel 7, 75 % | VI.20 |
| 98 | 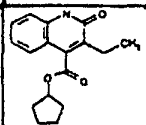 | 155 - 156 | analog Beispiel 7, 46 % | VI.3 |
| 99 | 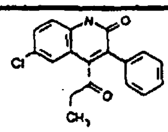 | 230 - 233 | analog Beispiel 16, 19 % | VI.2 |
| 100 | 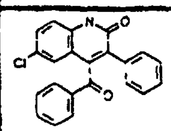 | 266 - 267 | siehe Beispiel 16, 13 % | VI.2 |
| 101 | 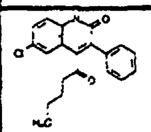 | 180 - 182 | analog Beispiel 16, 14 % | VI.2 |

"Advances in Heterocyclic Chemistry", A.R. Katritzky (Ed.), Academic Press, San Diego ).

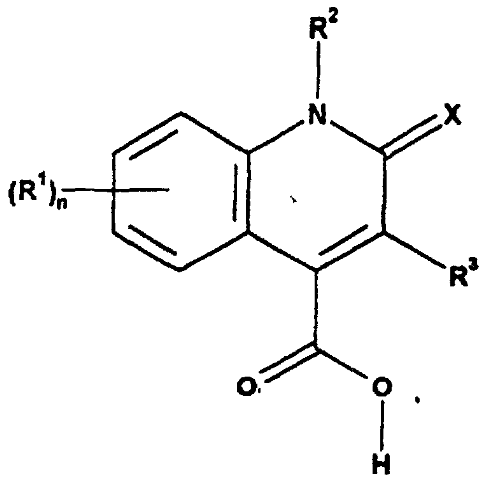

VI

sowie deren tautomere Form, der allgemeinen Formel VIa,

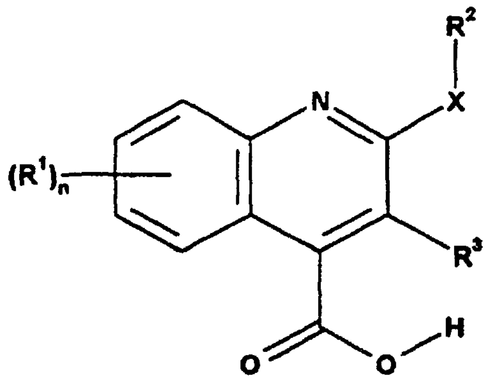

VIa

Tabelle 4 gibt eine Übersicht aller bislang synthetisierten Chinolin-4-carbonsäuren.

Tabelle 4

| Bezeichnung | R(1) | R(2) | R(3) | X | Herstellungsmethode Ausbeute |
|---|---|---|---|---|---|
| VI.1 | H | H | phenyl | O | siehe Beispiel 6; 74 % |
| VI.2 | 6-Cl | H | phenyl | O | analog Beispiel 6, 43 % |
| VI.3 | H | H | ethyl | O | analog Beispiel 9, 36 % |
| VI.4 | H | H | isobutyl | O | analog Beispiel 9, 48 % |
| VI.5 | 6-F | H | phenyl | O | analog Beispiel 6, 91 % |
| VI.6 | H | H | ethenyl | O | analog Beispiel 1, 16 % |
| VI.7 | 6-F | H | 2,6-$F_2$-phenyl | O | analoge Beispiel 6, 85 % |
| VI.8 | 6-Cl | H | benzyl | O | analog Beispiel 1, 13 % |
| VI.9 | 6-$CF_3O$ | H | phenyl | O | analog Beispiel 6, 99 % |
| VI.10 | H | H | methyl | O | analog Beispiel 1, 17 % |
| VI.11 | H | H | isopropyl | O | analog Beispiel 1, 26 % |
| VI.12 | H | H | 2-Cl-phenyl | O | analog Beispiel 6, 80 % |
| VI.13 | 6-Cl | H | methyl | O | siehe Beispiel 1; 74 % |

Tabelle 4 (fortgesetzt)

| Bezeichnung | R(1) | R(2) | R(3) | X | Herstellungsmethode Ausbeute |
|---|---|---|---|---|---|
| VI.14 | 6-Cl | H | ethyl | O | siehe Beispiel 9, 42 % |
| VI.15 | 6-Cl | H | 4-Cl-phenyl | O | analog Beispiel 6, 75 % |
| VI.16 | 6-Cl | H | 2-$CH_3O$-phenyl | O | analog Beispiel 6, 99 % |
| VI.17 | 6-Cl | H | 3-$CH_3O$-phenyl | O | analog Beispiel 6, 47 % |
| VI-18 | 6-Cl | H | 4-$CH_3O$-phenyl | O | analog Beispiel 6, 67 % |
| VI.19 | 6-F | H | ethyl | O | analog Beispiel 9, 13 % |
| VI.20 | 6-Br | H | ethyl | O | analog BEispiel 1, 12 % |
| VI.21 | 6-$CH_3O$ | H | phenyl | O | analog Beispiel 6, 74 % |
| VI.22 | 6-Cl-8-$CH_3$ | H | $CH_3$ | O | analog Beispiel 1, 27 % |

Die als Edukte zur Herstellung der Chinolin-carbonsäuren verwendeten Indolderivate können nach den Methoden der Beispiele I - III hergestellt werden.

**Patentansprüche**

**1.** Verbindungen der Formel 1,

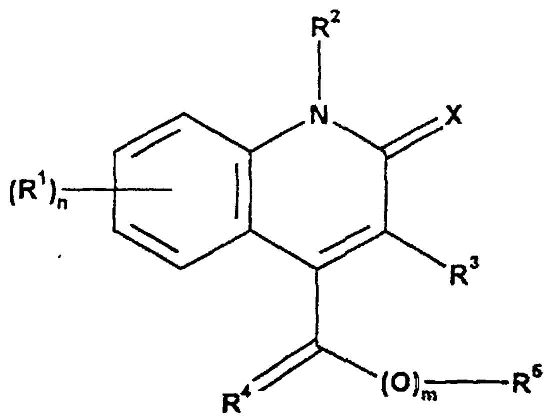

(I)

sowie deren tautomere Formen, der allgemeinen Formal Ia,

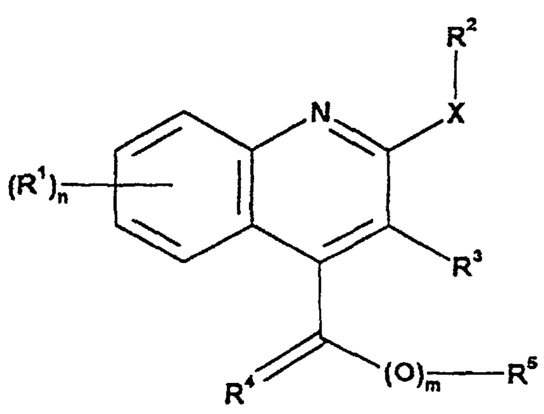

(Ia)

worin bedeuten: n
null,
eins,
zwei,
die einzelnen Substituenten $R^1$ unabhängig voneinander
Fluor, Chlor, Brom, Trifluormethyl, Trifluormethoxy, $(C_1-C_6)$-Alkyl, $(C_3-C_6)$-Cycloalkyl, $(C_1-C_6)$-Alkoxy, $(C_1-C_6)$-Alkoxy($(C_1-C_6)$-alkoxy), $(C_1-C_6)$-Alkylthio, $(C_1-C_6)$-Alkylsulfinyl, $(C_1-C_6)$-Acyl, $(C_1-C_6)$-Acyloxy, Carboxy, $(C_1-C_6)$-Alkyloxycarbonyl,
oder
einen gegebenenfalls mit bis zwei voneinander unabhängigen Resten $R^6$ substituierten Phenyl-, Phenoxy-, Phenoxycarbonyl-, Phenylthio-, Phenylsulfonyl-, Phenoxysulfonyl-, Phenylsulfonyloxy-, Phenylsulfonylamino-, Benzoylrest,
wobei $R^6$
Fluor, Chlor, Brom, Trifluormethyl, Trifluormethoxy, Azido, $(C_1-C_4)$-Alkyl, $(C_4-C_6)$-Cycloalkyl, $(C_1-C_4)$-Alkoxy, $(C_1-C_4)$-Alkylthio sein kann,
X Sauerstoff, Schwefel oder substituierten Stickstoff N-$R^2$, N-O-$R^2$, worin $R^2$ die unten gegebenen Bedeutungen haben kann,
und $R^2$ Wasserstoff,
$(C_1-C_4)$-Alkyl, gegebenenfalls substituiert mit Fluor, Chlor, Brom, $(C_1-C_3)$-Acyloxy, Benzoyloxy, Benzyloxy, Phenoxy, $(C_1-C_3)$-Alkoxy, Phenylsulfonyl, Oxo, Carboxy, Carbamoyl;
$(C_2-C_6)$-Alkenyl, gegebenenfalls substituiert mit Fluor, Chlor, Brom, $(C_1-C_3)$-Acyloxy, Benzoyloxy, Benzyloxy, Phenoxy, $(C_1-C_3)$-Alkoxy, Phenylsulfonyl, Oxo, Carboxy, Carbamoyl;
und
$R^3$

> $(C_1-C_8)$-Alkyl, gegebenenfalls substituiert mit Fluor, Chlor, Hydroxy, Amino, $(C_1-C_4)$-Acyloxy, Benzoyloxy, Benzyloxy, Phenoxy, $(C_1-C_4)$-Alkoxy, Carboxy;
> $(C_2-C_6)$-Alkenyl, gegebenenfalls substituiert mit Fluor, Chlor, Hydroxy, Amino, $(C_1-C_4)$-Acyloxy, Benzoyloxy, Benzyloxy, Phenoxy, $(C_1-C_4)$-Alkoxy, Carboxy;
> $(C_3-C_6)$-Cycloalkyl, gegebenenfalls substituiert ist Fluor, Chlor, Hydroxy,
> Amino, $(C_1-C_4)$-Acyloxy, Benzoyloxy, Benzyloxy, Phenoxy, $(C_1-C_4)$-Alkoxy, Carboxy;
> $(C_3-C_6)$-Cycloalkenyl, gegebenenfalls substituiert ist Fluor, Chlor, Hydroxy, Amino, $(C_1-C_4)$-Acyloxy, Benzoyloxy, Benzyloxy, Phenoxy, $(C_1-C_4)$-Alkoxy, Carboxy;

oder unsubstituiertes oder mit bis zu 2 voneinander unabhängigen Resten $R^6$ substituiertes Aryl, Aryl-$(C_1-C_2)$-alkyl bedeuten, wobei $R^6$ wie oben definiert ist,
und $R^4$ Sauerstoff,
und m gleich 0 oder 1,
und $R^5$

> $(C_1-C_8)$-Alkyl, gegebenenfalls substituiert mit Fluor, Chlor, Brom, Cyano, Amino, Hydroxy, $(C_1-C_4)$-Acyloxy, Benzoyloxy, Benzyloxy, Phenoxy, $(C_1-C_4)$-Alkoxy, Oxo, Carboxy, Carbamoyl;
> $(C_2-C_8)$-Alkenyl, gegebenenfalls substituiert mit Fluor, Chlor, Brom, Jod, Cyano, Amino, Hydroxy, $(C_1-C_4)$-Acyloxy, Benzoyloxy, Benzyloxy, Phenoxy, $(C_1-C_4)$-Alkoxy, Oxo, Carboxy, Carbamoyl;
> $(C_2-C_6)$-Alkinyl, gegebenenfalls substituiert mit Fluor, Chlor, Brom, Cyano, Amino, Hydroxy, $(C_1-C_4)$-Acyloxy, Benzoyloxy, Benzyloxy, Phenoxy, $(C_1-C_4)$-Alkoxy, Oxo, Carboxy, Carbamoyl;
> $(C_3-C_6)$-Cycloalkyl, gegebenenfalls substituiert mit Fluor, Chlor, Brom, Cyano, Amino, Hydroxy, $(C_1-C_4)$-Acyloxy, Benzoyloxy, Benzyloxy, Phenoxy, $(C_1-C_4)$-Alkoxy, Oxo, Carboxy, Carbamoyl;
> $(C_3-C_6)$-Cycloalkenyl, gegebenenfalls substituiert mit Fluor, Chlor, Brom, Amino, Hydroxy, $(C_1-C_4)$-Acyloxy, Benzoyloxy, Benzyloxy, Phenoxy, $(C_1-C_4)$-Alkoxy;
> $(C_3-C_6)$-(Cycloalkyl)-($(C_1-C_2)$-alkyl), gegebenenfalls substituiert mit Fluor, Chlor, Brom, Amino, Hydroxy, $(C_1-C_4)$-Acyloxy, Benzoyloxy, Benzyloxy, Phenoxy;
> $(C_3-C_6)$-(Cycloalkenyl)-($(C_1-C_2)$-alkyl), gegebenenfalls substituiert mit Fluor, Chlor, Brom, Amino, Hydroxy, $(C_1-C_4)$-Acyloxy, Benzoyloxy, Benzyloxy, Phenoxy;

oder unsubstituiertes oder mit bis zu zwei voneinander unabhängigen Resten $R^6$ substituiertes Aryl, Aryl-$(C_1-C_4)$-alkyl, Aryl-$(C_1-C_4)$-alkenyl, wobei $R^6$ wie oben definiert ist;
oder unsubstituiertes oder mit bis zu drei voneinander unabhängigen Resten $R^6$ substituiertes Heteroaryl, Hete-

roaryl-($C_1$-$C_4$)-alkyl,

deren optische Isomere, Diastereomere in reiner Form oder in Form ihrer Mischungen und deren Additionssalze und Prodrugs,

mit Ausnahme der Verbindungen der Formel I, in denen gleichzeitig die Reste $R^1$,$R^2$, $R^3$, $R^4$, $R^5$, X und m die folgenden Bedeutungen aufweisen:

$R^2$ gleich Wasserstoff,
$R^3$ gleich unsubstituiertes oder wie oben angegeben substituiertes Phenyl,
X gleich Sauerstoff,
$R^4$ gleich Sauerstoff,
m gleich 1 und
$R^5$ gleich $C_1$-$C_6$-Alkyl

weiterhin mit Ausnahme der Verbindungen der Formel I, in denen gleichzeitig die Reste $R^2$, $R^3$, $R^4$, $R^5$, X und m die folgenden Bedeutungen aufweisen:

$R^2$ gleich Wasserstoff,
$R^3$ gleich unsubstituiertes oder wie oben angegeben substituiertes Phenyl,
X gleich Sauerstoff,
$R^4$ gleich Sauerstoff,
m gleich O und
$R^5$ wie oben definiert.

**2.** Verbindungen der Formeln I oder Ia gemäß Anspruch 1, worin bedeuten
n
null,
eins,
der Substituenten $R^1$
Fluor, Chlor, Brom, Trifluormethyl, Trifluormethoxy, ($C_5$-$C_6$)-Cycloalkyl, ($C_1$-$C_4$)-Alkoxy, ($C_1$-$C_4$)-Alkoxy(($C_1$-$C_4$)-alkoxy), ($C_1$-$C_4$)-Alkylthio, ($C_1$-$C_4$)-Acyl,
oder
einen gegebenenfalls mit einem Rest $R^6$ substituierten Phenyl-, Phenoxy-, Benzoylrest,
wobei $R^6$
Fluor, Chlor, Brom, Trifluormethyl, Trifluormethoxy, ($C_1$-$C_4$)-Alkyl, ($C_1$-$C_4$)-Alkoxy, ($C_1$-$C_4$)-Alkylthio, sein kann,
X Sauerstoff oder Schwefel,
und $R^2$ Wasserstoff,
und
$R^3$

($C_1$-$C_6$)-Alkyl;
($C_2$-$C_6$)-Alkenyl;
($C_3$-$C_6$)-Cycloalkyl;

oder unsubstituiertes oder mit bis zu 2 voneinander unabhängigen Resten $R^6$ substituiertes Phenyl, Benzyl, wobei $R^6$ wie oben definiert ist,
und $R^4$ Sauerstoff,
und m gleich 1,
und $R^5$

($C_1$-$C_6$)-Alkyl, gegebenenfalls substituiert mit Fluor, Chlor, Brom, Cyano, Amino, Hydroxy, ($C_1$-$C_4$)-Acyloxy, Benzoyloxy, Benzyloxy, Phenoxy, ($C_1$-$C_4$)-Alkoxy;

($C_2$-$C_6$)-Alkenyl, gegebenenfalls substituiert mit Fluor, Chlor, Brom, Amino, Hydroxy, ($C_1$-$C_4$)-Acyloxy, Carboxy;

($C_3$-$C_6$)-Cycloalkyl;

($C_3$-$C_6$)-Cycloalkenyl;

$(C_3\text{-}C_6)$-(Cycloalkyl)-($(C_1\text{-}C_2)$-alkyl);

oder unsubstituiertes oder mit bis zu zwei voneinander unabhängigen Resten $R^6$ substituiertes Aryl, Aryl-$(C_1\text{-}C_4)$-alkyl, wobei $R^6$ wie oben definiert ist.

**3.** Verfahren zur Herstellung von Verbindungen gemäß einem oder mehreren der Ansprüche 1-2, **dadurch gekennzeichnet, daß** man

A) zur Herstellung von Verbindungen der Formel I oder Ia, worin X und $R^4$ Sauerstoff, m gleich 0 oder 1, mit den übrigen Resten wie in den Ansprüchen 1-2 definiert, eine Verbindung der Formel II, wobei A eine Abgangsgruppe, vorzugsweise ein Halogenatom darstellt, umsetzt mit einer Verbindung der Formel III, wobei M Wasserstoff,ein Metallatom oder ein Metallatomäquivalent, vorzugsweise ein Alkali- oder Erdalkalimetall darstellt,

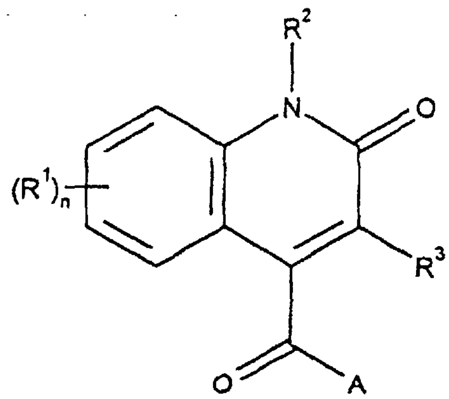

II

$R^5 — (O)_m — M$

III,

oder daß man

B) Verbindungen der Formel I mit X gleich Sauerstoff, und $R^1$ bis $R^5$ wie oben definiert, umsetzt mit einem Schwelfelungsreagenz zu Verbindungen der Formel I mit X gleich Schwefel und $R^1$ bis $R^5$ wie in den Ansprüchen 1-2 definiert, oder daß man
oder daß man

C) eine Verbindung der Formel I, mit $R^1$ - $R^5$ wie in den Ansprüchen 1-2 definiert und X ein Sauerstoff- oder Schwefelatom ist, umsetzt mit einer Verbindung der Formel IV oder IVa

$R^2$-NH2

(IV)

oder

$R^2\text{-}O\text{-}NH_2$

(IVa)

zu Derivaten der Formel I mit $R^1$ - $R^5$ wie in den Ansprüchen 1-2 definiert und X gleich N-$R^2$ oder N-O-$R^2$ oder daß man

D) eine Verbindung der Formel II mit A gleich Chlor und $R^1$ bis $R^5$ wie in den Ansprüchen 1-2 definiert umsetzt mit einem Reduktionsmittel zu Verbindungen der Formel I mit $R^1$ bis $R^3$ wie oben definiert, wobei $R^4$ gemeinsam mit dem verknüpfenden C-Atom eine $CH_2$-Gruppe bildet und $(O)_m$- $R^5$ gleich Hydroxy ist,

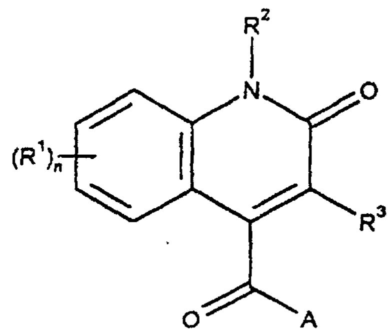

II

oder daß man

E) eine Verbindung der Formel I oder Ia mit $R^2$ gleich Wasserstoff und $R^1$, $R^3$, $R^4$ und $R^5$ wie in den Ansprüchen 1-2 definiert, umsetzt mit einer Verbindung der Formel V

R(2)-B (V),

wobei B eine Abgangsgruppe ist, zu Derivaten der Formel I, bei denen für $R^2$ die in den Ansprüchen 1-2 für die Verbindungen der Formel I und Ia beschriebenen Definitionen mit Ausnahme von $R^2$ gleich Wasserstoff gelten, umsetzt.

**4.** Verwendung von Verbindungen der Formeln I bzw. Ia gemäß einem oder mehreren der Ansprüche 1-2 zur Anwendung als Arzneimittel.

**5.** Arzneimittel, enthaltend eine wirksame Menge mindestens einer Verbindung Formeln I bzw. Ia gemäß einem oder mehreren der Ansprüche 1-2.

**6.** Verfahren zur Herstellung eines Arzneimittels gemäß Anspruch 5, **dadurch gekennzeichnet, daß** eine wirksame Menge einer Verbindung der Formeln I bzw. Ia mit üblichen pharmazeutischen Hilfsstoffen in eine geeignete Darreichungsform gebracht wird.

**7.** Verwendung von Verbindungen gemäß den Ansprüchen 1 oder 2 zur Herstellung von Arzneimitteln zur Behandlung von Krankheiten, die durch Retrovirusinfektionen hervorgerufen werden.

**Claims**

**1.** A compound of the formula I,

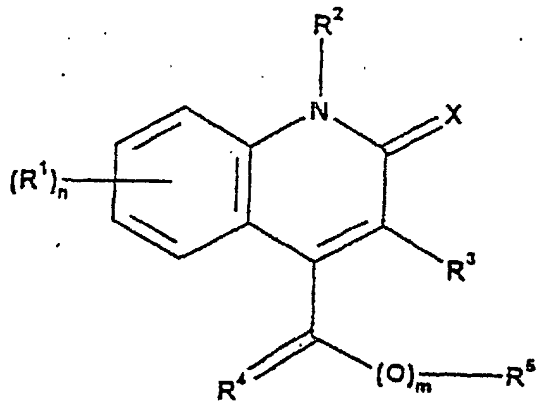

(I)

5 and tautomeric forms thereof of the formula Ia,

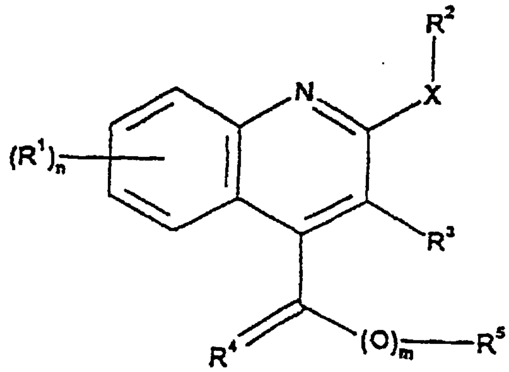

(Ia)

in which:

n is
zero,
one,
two,
the individual substituents $R^1$ are, independently of each other, fluorine, chlorine, bromine, trifluoromethyl, trifluoromethoxy, $(C_1-C_6)$-alkyl, $(C_3-C_6)$-cycloalkyl, $(C_1-C_6)$-alkoxy, $(C_1-C_6)$-alkoxy($(C_1-C_6)$-alkoxy), $(C_1-C_6)$-alkylthio, $(C_1-C_6)$-alkylsulfinyl, $(C_1-C_6)$-acyl, $(C_1-C_6)$-acyloxy, carboxyl or $(C_1-C_6)$-alkyloxycarbonyl,
or
a phenyl, phenoxy, phenoxycarbonyl, phenylthio, phenylsulfonyl, phenoxysulfonyl, phenylsulfonyloxy, phenylsulfonylamino or benzoyl radical which is optionally substituted by up to two radicals $R^6$ which are independent of each other,
where $R^6$ can be
fluorine, chlorine, bromine, trifluoromethyl, trifluoromethoxy, azido, $(C_1-C_4)$-alkyl, $(C_4-C_6)$-cycloalkyl, $(C_1-C_4)$-alkoxy or $(C_1-C_4)$-alkylthio,
X is oxygen, sulfur or substituted nitrogen N-$R^2$ or N-O-$R^2$, in which $R^2$ can have the meanings given below,
and $R^2$ is hydrogen,
$(C_1-C_4)$-alkyl which is optionally substituted by fluorine, chlorine, bromine, $(C_1-C_3)$-acyloxy, benzoyloxy, benzyloxy, phenoxy, $(C_1-C_3)$-alkoxy, phenylsulfonyl, oxo, carboxyl or carbamoyl;
or $(C_2-C_6)$-alkenyl which is optionally substituted by fluorine, chlorine, bromine, $(C_1-C_3)$-acyloxy, benzoyloxy, benzyloxy, phenoxy, $(C_1-C_3)$-alkoxy, phenylsulfonyl, oxo, carboxyl or carbamoyl;
and
$R^3$ is
$(C_1-C_8)$-alkyl which is optionally substituted by fluorine, chlorine, hydroxyl, amino, $(C_1-C_4)$-acyloxy, benzoyloxy, benzyloxy, phenoxy, $(C_1-C_4)$-alkoxy or carboxyl;
$(C_2-C_6)$-alkenyl which is optionally substituted by fluorine, chlorine, hydroxyl, amino, $(C_1-C_4)$-acyloxy, benzoy-

loxy, benzyloxy, phenoxy, $(C_1-C_4)$-alkoxy or carboxyl;
$(C_3-C_6)$-cycloalkyl which is optionally substituted by fluorine, chlorine, hydroxyl, amino, $(C_1-C_4)$-acyloxy, benzoyloxy, benzyloxy, phenoxy, $(C_1-C_4)$-alkoxy or carboxyl;
$(C_3-C_6)$-cycloalkenyl which is optionally substituted by fluorine, chlorine, hydroxyl, amino, $(C_1-C_4)$-acyloxy, benzoyloxy, benzyloxy, phenoxy, $(C_1-C_4)$-alkoxy or carboxyl;
or aryl or aryl-$(C_1-C_2)$-alkyl which is unsubstituted or substituted by up to 2 radicals $R^6$ which are independent of each other, where $R^6$ is defined as above,
and $R^4$ is oxygen,
and m is 0 or 1,
and $R^5$ is
$(C_1-C_8)$-alkyl which is optionally substituted by fluorine, chlorine, bromine, cyano, amino, hydroxyl, $(C_1-C_4)$-acyloxy, benzoyloxy, benzyloxy, phenoxy, $(C_1-C_4)$-alkoxy, oxo, carboxyl or carbamoyl;
$(C_2-C_8)$-alkenyl which is optionally substituted by fluorine, chlorine, bromine, iodine, cyano, amino, hydroxyl, $(C_1-C_4)$-acyloxy, benzoyloxy, benzyloxy, phenoxy, $(C_1-C_4)$-alkoxy, oxo, carboxyl or carbamoyl;
$(C_2-C_6)$-alkynyl which is optionally substituted by fluorine, chlorine, bromine, cyano, amino, hydroxyl, $(C_1-C_4)$-acyloxy, benzoyloxy, benzyloxy, phenoxy, $(C_1-C_4)$-alkoxy, oxo, carboxyl or carbamoyl;
$(C_3-C_6)$-cycloalkyl which is optionally substituted by fluorine, chlorine, bromine, cyano, amino, hydroxyl, $(C_1-C_4)$-acyloxy, benzoyloxy, benzyloxy, phenoxy, $(C_1-C_4)$-alkoxy, oxo, carboxyl or carbamoyl;
$(C_3-C_6)$-cycloalkenyl which is optionally substituted by fluorine, chlorine, bromine, amino, hydroxyl, $(C_1-C_4)$-acyloxy, benzoyloxy, benzyloxy, phenoxy or $(C_1-C_4)$-alkoxy;
$(C_3-C_6)$-(cycloalkyl)-($(C_1-C_2)$-alkyl) which is optionally substituted by fluorine, chlorine, bromine, amino, hydroxyl, $(C_1-C_4)$-acyloxy, benzoyloxy, benzyloxy or phenoxy;
$(C_3-C_6)$-(cycloalkenyl)-($(C_1-C_2)$-alkyl) which is optionally substituted by fluorine, chlorine, bromine, amino, hydroxyl, $(C_1-C_4)$-acyloxy, benzoyloxy, benzyloxy or phenoxy;
or aryl, aryl-$(C_1-C_4)$-alkyl or aryl-$(C_1-C_4)$-alkenyl which is unsubstituted or substituted by up to two radicals $R^6$ which are independent of each other, where $R^6$ is defined as above;
or heteroaryl or heteroaryl-$(C_1-C_4)$-alkyl which is unsubstituted or substituted by up to three radicals $R^6$ which are independent of each other,

their optical isomers, diastereomers in pure form or in the form of their mixtures, and their addition salts and prodrugs,

with the exception of the compounds of the formula I in which the radicals $R^2$, $R^3$, $R^4$, $R^5$, X and m simultaneously have the following meanings:

$R^2$ is hydrogen,
$R^3$ is unsubstituted phenyl or phenyl which is substituted as indicated above,
X is oxygen,
$R^4$ is oxygen,
m is 1, and
$R^5$ is $C_1-C_6$-alkyl

and, furthermore, with the exception of the compounds of the formula I in which the radicals $R^2$, $R^3$, $R^4$, $R^5$, X and m simultaneously have the following meanings:

$R^2$ is hydrogen,
$R^3$ is unsubstituted phenyl or phenyl which is substituted as indicated above,
X is oxygen,
$R^4$ is oxygen,
m is 0, and
$R^5$ is defined as above.

**2.** A compound of the formulae I or Ia as claimed in claim 1, in which n is
zero, or
one,
the substituents $R^1$ are
fluorine, chlorine, bromine, trifluoromethyl, trifluoromethoxy, $(C_5-C_6)$-cycloalkyl, $(C_1-C_4)$-alkoxy, $(C_1-C_4)$-alkoxy($(C_1-C_4)$-alkoxy), $(C_1-C_4)$-alkylthio or $(C_1-C_4)$-acyl,
or
a phenyl, phenoxy or benzoyl radical which is optionally substituted by a radical $R^6$,

where $R^6$ can be fluorine, chlorine, bromine, trifluoromethyl, trifluoromethoxy, $(C_1-C_4)$-alkyl, $(C_1-C_4)$-alkoxy or $(C_1-C_4)$-alkylthio, X is oxygen or sulfur, and $R^2$ is hydrogen, and $R^3$ is

$(C_1-C_6)$-alkyl;
$(C_2-C_6)$-alkenyl;
$(C_3-C_6)$-cycloalkyl;
or phenyl or benzyl which is unsubstituted or substituted by up to 2 radicals $R^6$ which are independent of each other, where $R^6$ is defined as above,
and $R^4$ is oxygen,
and m is 1,
and $R^5$ is
$(C_1-C_6)$-alkyl which is optionally substituted by fluorine, chlorine, bromine, cyano, amino, hydroxyl, $(C_1-C_4)$-acyloxy, benzoyloxy, benzyloxy, phenoxy or $(C_1-C_4)$-alkoxy;
$(C_2-C_6)$-alkenyl which is optionally substituted by fluorine, chlorine, bromine, amino, hydroxyl, $(C_1-C_4)$-acyloxy or carboxyl;
$(C_3-C_6)$-cycloalkyl;
$(C_3-C_6)$-cycloalkenyl;
$(C_3-C_6)$-(cycloalkyl)-($(C_1-C_2)$-alkyl);
or aryl or aryl-$(C_1C_4)$-alkyl which is unsubstituted or substituted by up to two radicals $R^6$ which are independent of each other, where $R^6$ is defined as above.

**3.** A process for preparing compounds as claimed in one or more of claims 1-2, which comprises

A) for preparing compounds of the formula (I) or (Ia) in which X and $R^4$ are oxygen, and m is 0 or 1, with the other radicals being defined as in claims 1-2, reacting a compound of the formula II, where A is a leaving group, preferably a halogen atom, with a compound of the formula III, where M is hydrogen, a metal atom or a metal atom equivalent, preferably an alkali metal or alkaline earth metal,

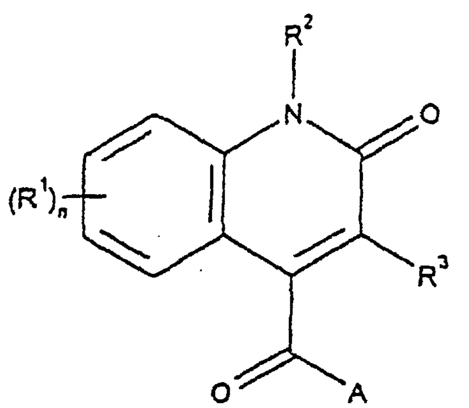

II

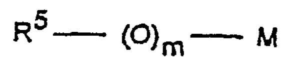

III,

or which comprises

B) reacting compounds of the formula I in which X is oxygen, and $R^1$ to $R^5$ are defined as above, with a sulfurating reagent to give compounds of the formula I in which X is sulfur and $R^1$ to $R^5$ are defined as in claims 1-2, or which comprises

C) reacting a compound of the formula I in which $R^1$-$R^5$ are defined as in claims 1-2 and X is an oxygen atom or a sulfur atom, with a compound of the formula IV or IVa

$$R^2\text{-}NH_2$$

(IV)

or

$$R^2\text{-}O\text{-}NH_2$$

(IVa)

to give derivatives of the formula I in which $R^1$-$R^5$ are defined as in claims 1-2 and X is N-$R^2$ or N-O-$R^2$ or which comprises

D) reacting a compound of the formula II in which A is chlorine and $R^1$ to $R^5$ are defined as in claims 1-2 with a reducing agent to give compounds of the formula I in which $R^1$ to $R^3$ are defined as above, where $R^4$, together with the linking C atom, forms a $CH_2$ group and $(O)_m$-$R^5$ is hydroxyl,

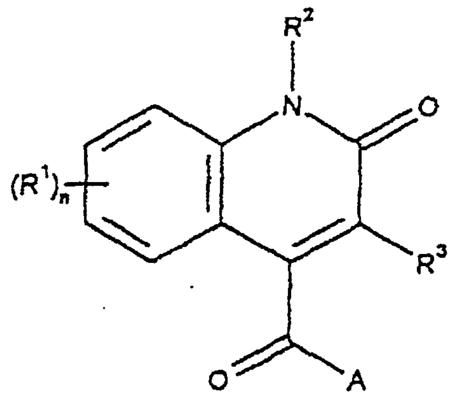

II

or which comprises

E) reacting a compound of the formula I or Ia in which $R^2$ is hydrogen and $R^1$, $R^3$, $R^4$ and $R^5$ are defined as in claims 1-2, with a compound of the formula V

R(2)-B (V)

where B is a leaving group, to give derivatives of the formula I in which the definitions described in claims 1-2 for the compounds of the formulae I and Ia apply for $R^2$ with the exception of $R^2$ being hydrogen.

**4.** The use of compounds of the formulae I and Ia as claimed in one or more of claims 1-2 for employment as pharmaceuticals.

**5.** A pharmaceutical which comprises an effective quantity of at least one compound of the formulae I and Ia as

claimed in one or more of claims 1-2.

6. A process for producing a pharmaceutical as claimed in claim 5, which comprises bringing an effective quantity of a compound of the formulae I and Ia, together with customary pharmaceutical auxiliary substances, into a suitable form for administration.

7. The use of compounds as claimed in claims 1 or 2 for producing pharmaceuticals for treating diseases which are elicited by retrovirus infections.

**Revendications**

1. Composés de formule I

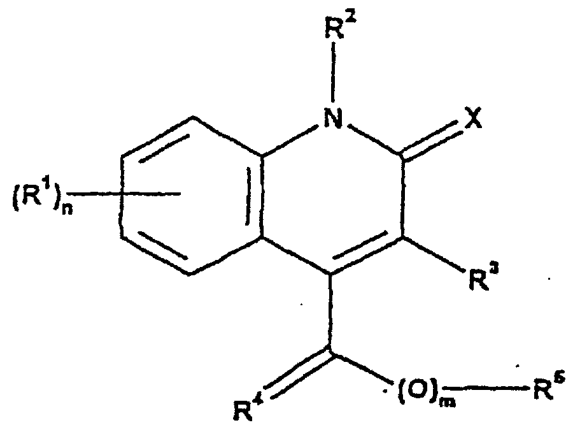

(I)

ainsi leurs formes tautomères de formule générale Ia

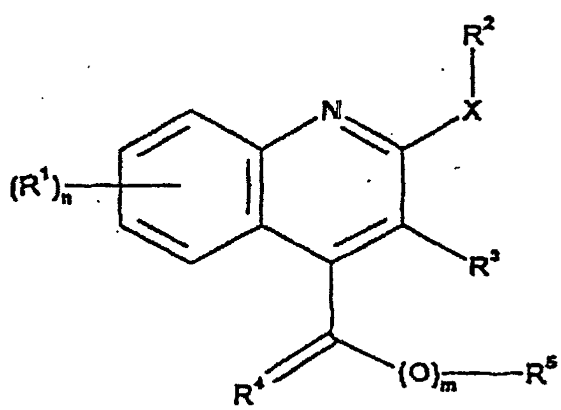

(Ia)

où

n vaut zéro, un, deux,
les différents substituants

$R^1$ représentent, indépendamment les uns des autres, des atomes de fluor, de chlore, de brome, des groupes trifluorométhyle, trifluorométhoxy, alkyle en $C_1$-$C_6$, cycloalkyle en $C_3$-$C_6$, alkoxy en $C_1$-$C_6$, (alkoxy en $C_1$-$C_6$)-(alkoxy en $C_1$-$C_6$), (alkyl en $C_1$-$C_6$)thio, (alkyl en $C_1$- $C_6$)sulfinyle, acyle en $C_1$-$C_6$, acyloxy en $C_1$-$C_6$, carboxy, (alkyloxy en $C_1$-$C_6$)carbonyle, ou
un reste phényle, phénoxy, phénoxycarbonyle, phénylthio, phénylsulfonyle, phénoxysulfonyle, phénylsulfonyloxy, phénylsulfonylamino, benzoyle, éventuellement substitué par jusqu'à deux restes $R^6$ indé-

pendants l'un de l'autre,

$R^6$ pouvant représenter

des atomes de fluor, de chlore, de brome, des groupes trifluorométhyle, trifluorométhoxy, azido, alkyle en $C_1$-$C_4$, cycloalkyle en $C_4$-$C_6$, alkoxy en $C_1$-$C_4$, (alkyl en $C_1$-$C_4$)thio,

X représente

un atome d'oxygène, un atome de soufre ou un atome d'azote substitué N-$R^2$, N-O-$R^2$, où $R^2$ peut posséder les significations données ci-dessous, et

$R^2$ représente un atome d'hydrogène,

un groupe alkyle en $C_1$-$C_4$, éventuellement substitué par des substituants fluor, chlore, brome, acyloxy en $C_1$-$C_3$, benzoyloxy, benzyloxy, phénoxy, alkoxy en $C_1$-$C_3$, phénylsulfonyle, oxo, carboxy, carbamoyle ;

un groupe alcényle en $C_2$-$C_6$, éventuellement substitué par des substituants fluor, chlore, brome, acyloxy en $C_1$-$C_3$, benzoyloxy, benzyloxy, phénoxy, alkoxy en $C_1$-$C_3$, phénylsulfonyle, oxo, carboxy, carbamoyle ; et

$R^3$ représente

un groupe alkyle en $C_1$-$C_8$, éventuellement substitué par des substituants fluor, chlore, hydroxy, amino, acyloxy en $C_1$-$C_4$, benzoyloxy, benzyloxy, phénoxy, alkoxy en $C_1$-$C_4$, carboxy ;

un groupe alcényle en $C_2$-$C_6$, éventuellement substitué par des substituants fluor, chlore, hydroxy, amino, acyloxy en $C_1$-$C_4$, benzoyloxy, benzyloxy, phénoxy, alkoxy en $C_1$-$C_4$, carboxy ;

un groupe cycloalkyle en $C_3$-$C_6$, éventuellement substitué par des substituants fluor, chlore, hydroxy, amino, acyloxy en $C_1$-$C_4$, benzoyloxy, benzyloxy, phénoxy, alkoxy en $C_1$-$C_4$, carboxy ;

un groupe cycloalcényle en $C_3$-$C_6$, éventuellement substitué par des substituants fluor, chlore, hydroxy, amino, acyloxy en $C_1$-$C_4$, benzoyloxy, benzyloxy, phénoxy, alkoxy en $C_1$-$C_4$, carboxy ; ou

un groupe aryle non substitué ou substitué par jusqu'à deux restes $R^6$ indépendants, un groupe arylalkyle en $C_1$-$C_2$, $R^6$ étant défini ci-dessus, et

$R^4$ représente un atome d'oxygène, et

m vaut 0 ou 1, et

$R^5$ représente

un groupe alkyle en $C_1$-$C_8$, éventuellement substitué par des substituants fluor, chlore, brome, cyano, amino, hydroxy, acyloxy en $C_1$-$C_4$, benzoyloxy, benzyloxy, phénoxy, alkoxy en $C_1$-$C_4$, oxo, carboxy, carbamoyle ;

un groupe alcényle en $C_2$-$C_8$, éventuellement substitué par des substituants fluor, chlore, brome, iode, cyano, amino, hydroxy, acyloxy en $C_1$-$C_4$, benzoyloxy, benzyloxy, phénoxy, alkoxy en $C_1$-$C_4$, oxo, carboxy, carbamoyle ;

un groupe alcynyle en $C_2$-$C_6$, éventuellement substitué par des substituants fluor, chlore, brome, cyano, amino, hydroxy, acyloxy en $C_1$-$C_4$, benzoyloxy, benzyloxy, phénoxy, alkoxy en $C_1$-$C_4$, oxo, carboxy, carbamoyle ;

un groupe cycloalkyle en $C_3$-$C_6$, éventuellement substitué par des substituants fluor, chlore, brome, cyano, amino, hydroxy, acyloxy en $C_1$-$C_4$, benzoyloxy, benzyloxy, phénoxy, alkoxy en $C_1$-$C_4$, oxo, carboxy, carbamoyle ;

un groupe cycloalcényle en $C_3$-$C_6$, éventuellement substitué par des substituants fluor, chlore, brome, amino, hydroxy, acyloxy en $C_1$-$C_4$, benzoyloxy, benzyloxy, phénoxy, alkoxy en $C_1$-$C_4$ ;

un groupe (cycloalkyl en $C_3$-$C_6$) - (alkyle en $C_1$-$C_2$) éventuellement substitué par des substituants fluor, chlore, brome, amino, hydroxy, acyloxy en $C_1$-$C_4$, benzoyloxy, benzyloxy, phénoxy ;

un groupe (cycloalcényl en $C_3$-$C_6$) - (alkyle en $C_1$ - $C_2$) éventuellement substitué par des substituants fluor, chlore, brome, amino, hydroxy, acyloxy en $C_1$-$C_4$, benzoyloxy, benzyloxy, phénoxy ;

ou un groupe aryle non substitué ou substitué par jusqu'à deux restes $R^6$ indépendants, des groupes arylalkyle en $C_1$-$C_4$, arylalcényle en $C_1$-$C_4$, $R^6$ étant défini comme ci-dessus ;

ou un groupe hétéroaryle non substitué ou substitué par jusqu'à trois restes $R^6$ indépendants, un groupe hétéroarylalkyle en $C_1$-$C_4$ ;

leurs isomères optiques, diastéréomères sous forme pure ou sous forme de leurs mélanges et leurs sels d'addition et des précurseurs de médicaments,

à l'exception des composés de formule I, dans lesquels les restes $R^1$, $R^2$, $R^3$, $R^4$, $R^5$, X et m présentent en même temps les significations suivantes :

$R^2$ représente un atome d'hydrogène,

$R^3$ représente

un groupe phényle non substitué ou substitué

comme indiqué ci-dessus,

X représente un atome d'oxygène,
$R^4$ représente un atome d'oxygène,
m vaut 1 et
$R^5$ représente
un groupe alkyle en $C_1$-$C_6$,

de plus, à l'exception des composés de formule I, dans lesquels les restes $R^2$, $R^3$, $R^4$, $R^5$, X et m présentent en même temps les significations suivantes :

$R^2$ représente un atome d'hydrogène,
$R^3$ représente

un groupe phényle non substitué ou substitué comme indiqué ci-dessus,

X représente un atome d'oxygène,
$R^4$ représente un atome d'oxygène,
m vaut 0 et
$R^5$ est défini comme ci-dessus.

**2.** Composés de formules I ou Ia selon revendication 1, où représentent

n vaut zéro, un,
les substituants
$R^1$ représentent
des atomes de fluor, de chlore, de brome, des groupes trifluorométhyle, trifluorométhoxy, cycloalkyle en $C_5$-$C_6$, alkoxy en $C_1$-$C_4$, (alkoxy en $C_1$-$C_4$)- (alkoxy en $C_1$-$C_4$), (alkyl en $C_1$-$C_4$)thio, acyle en $C_1$-$C_4$, ou
un reste phényle, phénoxy, benzoyle, éventuellement substitué par un reste $R^6$,
$R^6$ pouvant représenter
des atomes de fluor, de chlore, de brome, des groupes trifluorométhyle, trifluorométhoxy, alkyle en $C_1$-$C_4$, alkoxy en $C_1$-$C_4$, (alkyl en $C_1$-$C_4$)thio,
X représente
un atome d'oxygène ou un atome de soufre, et
$R^2$ représente un atome d'hydrogène, et
$R^3$ représente
un groupe alkyle en $C_1$-$C_6$, un groupe alcényle en $C_2$-$C_6$, un groupe cycloalkyle en $C_3$-$C_6$,
ou
un groupe phényle, un groupe benzyle non substitué ou substitué par jusqu'à deux restes $R^6$ indépendants, $R^6$ étant défini ci-dessus, et
$R^4$ représente un atome d'oxygène, et
m vaut 1, et
$R^5$ représente
un groupe alkyle en $C_1$-$C_6$, éventuellement substitué par des substituants fluor, chlore, brome, cyano, amino, hydroxy, acyloxy en $C_1$-$C_4$, benzoyloxy, benzyloxy, phénoxy, en $C_1$-$C_4$ ;
un groupe alcényle en $C_2$-$C_6$, éventuellement substitué par des substituants fluor, chlore, brome, amino, hydroxy, acyloxy en $C_1$-$C_4$, carboxy ;
un groupe cycloalkyle en $C_3$-$C_6$ ;
un groupe cycloalcényle en $C_3$-$C_6$ ;
un groupe (cycloalkyl en $C_3$-$C_6$) - (alkyle en $C_1$-$C_2$) ;

ou un groupe aryle non substitué ou substitué par
jusqu'à deux restes $R^6$ indépendants, un groupe arylalkyle en $C_1$-$C_4$, $R^6$ étant défini comme ci- dessus.

**3.** Procédé pour la préparation de composés selon une ou plusieurs des revendications 1-2, **caractérisé en ce que**

A) pour la préparation de composés de formule I ou Ia, où X et $R^4$ représentent un atome d'oxygène, m vaut 0 ou 1, avec les autres restes définis comme dans les revendications 1-2, on fait réagir un composé de formule

II, où A représente un groupe partant, de préférence un atome d'halogène, sur un composé de formule III, où M représente un atome d'hydrogène, un atome métallique ou un équivalent d'atome métallique, de préférence un métal alcalin ou alcalinoterreux,

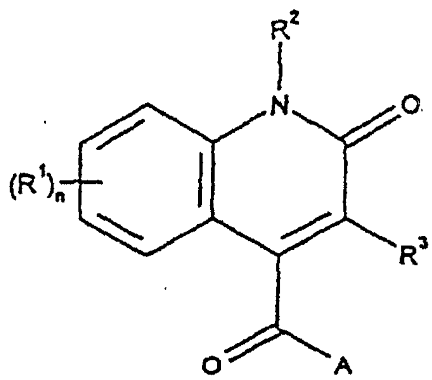

$R^5 — (O)_m — M$

II

III,

ou **en ce qu'**on fait réagir

B) des composés de formule I avec X représentant un atome d'oxygène, et $R^1$ à $R^5$ sont définis comme ci-dessus, sur un réactif de sulfuration pour obtenir des composés de formule I avec X représentant un atome de soufre et $R^1$ à $R^5$ étant définis dans les revendications 1-2,

ou **en ce qu'**on fait réagir

C) un composé de formule I, avec $R^1$ à $R^5$ définis comme dans les revendications 1-2 et X représentant un atome d'oxygène ou un atome de soufre, sur un composé de formule IV ou IVa

$$R^2-NH_2$$

(IV)

ou

$$R^2-O-NH_2$$

(IVa)

pour obtenir des dérivés de formule I avec $R^1$ à $R^5$ définis comme dans les revendications 1-2 et X représentant des groupes $N-R^2$ ou $N-O-R^2$,

ou **en ce qu'**on fait réagir

D) un composé de formule II avec A représentant un atome de chlore et $R^1$ à $R^5$ étant définis comme dans les revendications 1-2, sur un agent réducteur pour obtenir des composés de formule I avec $R^1$ à $R^3$ définis comme ci-dessus, $R^4$ formant, conjointement avec l'atome de carbone qui le relie, un groupe $CH_2$ et $(O)_m$-R représente un groupe hydroxy,

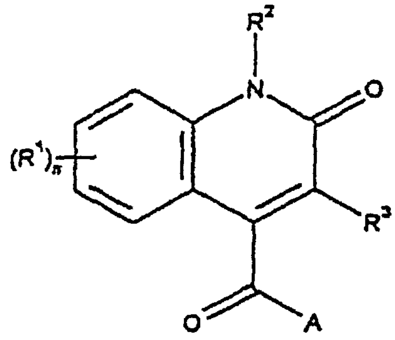

II

ou **en ce qu'**on fait réagir

E) un composé de formule I ou Ia avec $R^2$ représentant un atome d'hydrogène et $R^1$, $R^3$, $R^4$ et $R^5$ étant définis comme dans les revendications 1-2, sur un composé de formule V

R(2)-B (V),

B étant un groupe partant, pour obtenir des dérivés de formule I, dans lesquels les définitions données pour les composés de formule I et Ia s'appliquent à $R^2$, à l'exception de $R^2$ représentant un atome d'hydrogène.

4. Utilisation de composés de formules I ou Ia selon une ou plusieurs des revendications 1-2 pour l'application comme médicament.

5. Médicament contenant une quantité efficace d'au moins un composé de formules I ou Ia selon une ou plusieurs des revendications 1-2.

6. Procédé pour la préparation d'un médicament selon la revendication 5, **caractérisé en ce qu'**on met en forme d'administration appropriée une quantité efficace d'un composé de formules I ou Ia avec des adjuvants pharmaceutiques usuels.

7. Utilisation de composés selon l'une des revendications 1 ou 2 pour la préparation de médicaments pour le traitement de maladies provoquées par des infections rétrovirales.